# EUROPEAN PATENT SPECIFICATION

(11) **EP 4 286 359 B1**
(45) Date of publication and mention of the grant of the patent: **15.07.2026**
(21) Application number: 22745187.9
(22) Date of filing: 24.01.2022
(51) Int. Cl.: C07C 39/04, C07C 39/17, A61K 31/05, A61K 31/095, A61K 31/10, A61K 31/222, A61P 23/00, A61P 25/00, C07C 69/16, C07C 317/18, C07C 323/16

(54) **PHENOL DERIVATIVE AND APPLICATION THEREOF IN MEDICAMENTS**
PHENOLDERIVAT UND ANWENDUNG DAVON IN ARZNEIMITTELN
DÉRIVÉ DU PHÉNOL ET SON APPLICATION DANS DES MÉDICAMENTS

(30) Priority: 28.01.2021 CN 202110117767
(43) Date of publication of application: 06.12.2023
(73) Proprietor: Hinye Pharmaceutical Co., Ltd., Liuyang City Changsha, Hunan 410330 (CN)
(72) Inventor: LIU, Junhua, Changsha, Hunan 410330 (CN); LIANG, Xueping, Changsha, Hunan 410330 (CN); JIANG, Haigang, Changsha, Hunan 410330 (CN); WANG, Hengxin, Changsha, Hunan 410330 (CN); DENG, Lili, Changsha, Hunan 410330 (CN); SONG, Zhilin, Changsha, Hunan 410330 (CN)
(74) Representative: Pribic, Jelena
(86) International application number: PCT/CN2022/073472
(87) International publication number: WO 2022/161312

(56) References cited:
- WO-A2-03/026632
- WO-A2-03/026632
- CN-A- 105 384 608
- CN-A- 105 384 608
- CN-A- 112 245 426
- JAMES R ET AL: "SYNTHESIS, BIOLOGICAL EVALUATION, AND PRELIMINARY STRUCTURE-ACTIVITY CONSIDERATIONS OF A SERIES OF ALKYLPHENOLS AS INTRAVENOUS ANESTHETIC AGENTS", JOURNAL OF MEDICINAL CHEMISTRY, AMERICAN CHEMICAL SOCIETY, US, vol. 23, 1 January 1980 (1980-01-01), pages 1350 - 1357, XP002489327, ISSN: 0022-2623, DOI: 10.1021/JM00186A013
- JAMES, ROGER ET AL.: "Synthesis, Biological Evaluation, and Preliminary Structure-Activity Considerations of a Series of Alkylphenols as Intravenous Anesthetic Agents", JOURNAL OF MEDICINAL CHEMISTRY, vol. 23, no. 12, 31 December 1980 (1980-12-31), XP002489327, DOI: 10.1021/jm00186a013

## Description

### Technical Field

The present invention relates to the field of medicinal chemistry, in particular to a phenol derivative, a stereoisomer and pharmaceutically acceptable salt thereof, and the use of the compound of the present invention in the preparation of a medicament for treating central nervous system-related diseases.

### Background Art

A GABA_{A} receptor is a receptor of the chief inhibitory neurotransmitter in the central nervous system. The GABA_{A} receptor is composed of a pentamer of transmembrane polypeptide subunits, and 19 different subunits assemble into various GABA_{A} receptor subtypes. The GABA_{A} receptor is involved in pathogenesis, diagnosis and treatment of various diseases such as anesthesia, depression, anxiety, epilepsy, memory disorder and drug dependence. Accordingly, the GABA_{A} receptor has become a pharmacologically and clinically important drug target. Propofol and its derivatives represent a class of important GABA_{A}-targeting compounds.

Propofol can activate many GABA_{A} receptor subtypes, and is widely used for inducing and maintaining general anesthesia. Propofol shows remarkable pharmacokinetic and pharmacodynamic characteristics in that it rapidly takes effect, acts for a short period, and is quickly reversible. Upon intravenous administration, propofol in the blood rapidly enters hyperperfused areas such as heart, lung and liver, and its high liposolubility allows propofol to easily travel across the blood-brain barrier into the brain for general anesthesia.

With the in-depth clinical application of propofol, many of its limitations and disadvantages have been reported one after another. It has been reported that approximately 70% of patients on propofol injections feel certain pain or discomfort. It has been demonstrated that propofol can lower the systolic pressure, the diastolic pressure and the mean arterial pressure, and thus may clinically cause hypotension. Furthermore, respiratory depression is also an unneglectable risk upon use of propofol. These adverse effects have considerably impeded application of propofol in certain clinical cases, such as cardiovascular diseases, brain injury and chronic hypotension.

Fospropofol disodium is a water-soluble prodrug of propofol, and can be rapidly hydrolysed by alkaline phosphatase to release propofol, phosphate and formaldehyde. Although fospropofol disodium relieves pain at sites of intravenous propofol injection, it still poses risks of respiratory depression and adverse hemodynamic effects because it takes effect in the form of the active compound propofol. In addition, fospropofol disodium may also cause abnormal sensation and itching.

With regard to the limitations and disadvantages of propofol and fospropofol described above, there is a need for developing a novel GABA_{A} receptor agonist with better pharmacokinetic and pharmacodynamic characteristics and fewer side effects.

The patent US 20050032753 A1 describes a phenol derivative useful for anesthesia and sedation, wherein R¹ and R² are independently selected from C₁₋₈ alkyl and C₁₋₈ cycloalkyl; L is selected from a covalent bond or C₁₋₁₂ hydrocarbylene; and R³ is selected from -C(=O)ORₐ, wherein Rₐ is selected from C₁₋₁₂ hydrocarbylene.

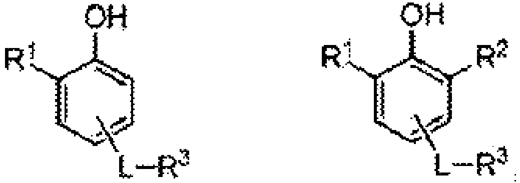

The patent further discloses the following formula, wherein R₄ is C₁-C₅ alkyl, C₂-C₅ alkenyl or C₂-C₅ alkynyl; R₅ is C₁-C₆ alkyl or C₃-C₈ cycloalkyl; R₆ is methyl; or R₅ and R₆ together with the carbon atoms to which they are attached form C₃₋₈ cycloalkyl; and Rₐ is C₁-C₈ alkyl, C₂-C₈ alkenyl, C₂-C₈ alkynyl or C₃-C₈ cycloalkyl. However, none of the compounds disclosed in the patent is included in the general structural formula of the present invention, and the patent does not disclose pharmacodynamic data for any specific compound. Those skilled in the art would not have been able to know from the patent whether different substituents result in differences in efficacy, and also would not have been motivated by the patent to make further improvement in a specific direction, to obtain specific compound structures with better efficacy. The compound of the patent greatly differs in structure from the compound of the present invention, and the specific descriptions in the patent are not considered as a part of the present invention.

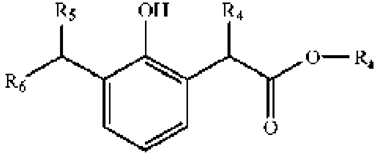

The patent CN 104507899 A discloses a phenol derivative, a preparation method therefor, and the use thereof in the central nervous field. The patent discloses the following general formula. The compound of the patent greatly differs in structure from the compound of the present invention, and the specific descriptions in the patent are not considered as a part of the present invention.

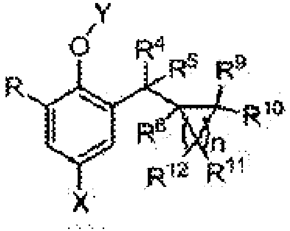

The patent CN 104507898 A discloses a phenol derivative, a preparation method therefor, and the use thereof in the central nervous field. The patent discloses the following general formula. The compound of the patent greatly differs in structure from the compound of the present invention, and the specific descriptions in the patent are not considered as a part of the present invention.

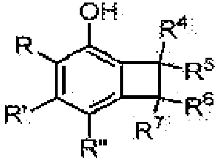

Patent Document WO03/026632A2 discloses a compound of formula (X), wherein Rₐ is (C₁-C₈)alkyl, (C₂-C₈)alkenyl, (C₂-C₈)alkynyl, or (C₃-C₈)cycloalkyl, and teaches the that this kind of the compound and pharmaceutical composition are useful for inducing and maintaining anesthesia or sedation in a mammal. However, none of the compounds disclosed in the patent is included in the general structural formula of the present invention, and the specific descriptions in the patent are not considered as a part of the present invention.

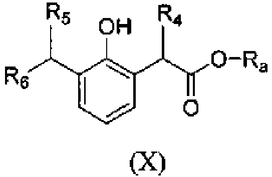

### Summary of the Invention

It is noted that the invention is set out in the appended set of claims.

An objective of the present invention is to provide a GABA_{A} receptor agonist phenol derivative that has a novel structure and better efficacy, can effectively reduce side effects, and is safer for clinical use, a stereoisomer thereof, and the use thereof in the central nervous field, thereby providing more and better choices for medicaments for inducing and/or maintaining anesthesia in animal or human bodies, facilitating sedation and hypnosis, and treating and/or preventing anxiety, nausea, vomiting, migraine, convulsion, epilepsy, neurodegenerative diseases, and central nervous system-related diseases.

In one aspect, the present invention provides a compound as represented by general formula (I), or a stereoisomer or pharmaceutically acceptable salt thereof: 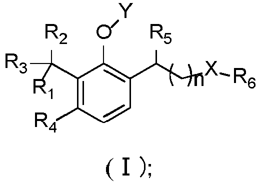 wherein
X is selected from 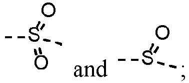
R₁ is selected from C₁₋₆ alkyl, C₁₋₆ alkene, C₁₋₆ alkyne, 3- to 6-membered heterocycloalkyl or 3- to 6-membered cycloalkyl, wherein the alkyl, alkene, alkyne, heterocycloalkyl and cycloalkyl may be optionally further substituted with one or more R;
R₂ and R₃ are independently selected from H, hydroxyl, F, C₁₋₆ alkyl, C₁₋₆ alkene, C₁₋₆ alkyne, 3- to 6-membered heterocycloalkyl, C₁₋₆ alkoxy, CN, NH₂ or 3- to 6-membered cycloalkyl, wherein the alkyl, alkene, alkyne, heterocycloalkyl, alkoxy and cycloalkyl may be optionally further substituted with one or more R;
or alternatively, R₂ and R₃ may form (=O);
R₄ is selected from H, F, Cl, Br, I, hydroxyl, C₁₋₆ alkyl, C₁₋₆ alkoxy, 3- to 5-membered cycloalkyl or 3- to 5-membered heterocyclyl;
or alternatively, R₁ and R₄ together with the atoms to which they are attached form 4- to 6-membered cycloalkyl or heterocyclyl fused to a benzene ring, wherein the cycloalkyl and heterocyclyl may be optionally further substituted with one or more R;
R₅ is selected from C₂-C₅ alkenyl, C₂-C₅ alkynyl, C₁₋₆ alkyl or 3- to 6-membered cycloalkyl, wherein the alkyl and cycloalkyl may be optionally further substituted with one or more R;
R₆ is selected from C₁₋₆ alkyl, 3- to 6-membered cycloalkyl or NHR₇, wherein the alkyl and cycloalkyl may be optionally further substituted with one or more R;
R₇ is selected from C₁₋₆ alkyl or cycloalkyl;
Y is selected from H, Na, K, 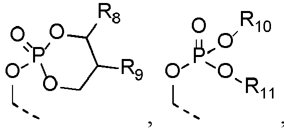 , -(CH₂) ₘCOOR₁₂, 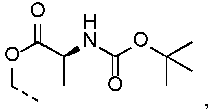 or C₁₋₁₀ alkyl, wherein the alkyl is optionally further substituted with one or more R;
R₈ and R₉ are each independently selected from H, C₁₋₆ alkyl, C₁₋₆ alkoxy, F, Cl, Br, I, hydroxyl, amino, cyano or carboxyl;
or alternatively, R₈ and R₉ together with the atoms to which they are attached form a 5- to 8-membered ring, wherein the 5- to 8-membered ring may contain 0 to 4 heteroatoms selected from N, O or S;
R₁₀ and R₁₁ are each independently selected from H, C₁₋₆ alkyl, an alkaline metal ion, an alkaline earth metal ion, a protonated amine or a protonated amino acid, wherein the alkaline metal ion is selected from Na⁺, K⁺ or Li⁺, the alkaline earth metal ion is selected from Be²⁺, Mg²⁺ or Ca²⁺, the amine is selected from trometamol, triethanolamine, ethanolamine, triethylamine or N-methylglucosamine, and the amino acid is selected from arginine or lysine;
R₁₂ is independently selected from H, C₁₋₆ alkyl, 3- to 8-membered cycloalkyl or 4- to 8-membered heterocyclyl, wherein the alkyl, cycloalkyl and heterocyclyl may be optionally further substituted with one or more R;
R is selected from F, Cl, Br, I, deuterium, hydroxyl, carboxyl, CN, NH₂, C₁₋₆ alkyl, C₁₋₆ alkoxy, 3- to 5-membered cycloalkyl or 3- to 5-membered heterocyclyl;
n is selected from 0, 1, 2 or 3; and
m is selected from 0, 1, 2, 3 or 4.

In some preferred embodiments provided by the present invention, the compound is selected from a compound as represented by general formula ( II ): 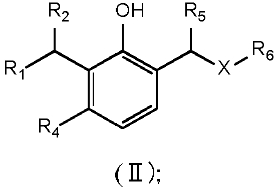 wherein X is selected from

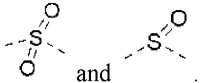

In another preferred embodiment provided by the present invention, the compound is selected from a compound as represented by general formula (III): 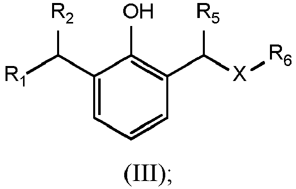 wherein
X is selected from 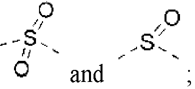 and
R₁, R₂, R₅ and R₆ are each independently selected from C₁₋₆ alkyl or 3- to 6-membered cycloalkyl.

Preferably,
R₁ is selected from C₁₋₆ alkyl or 3- to 6-membered cycloalkyl;
R₂ is selected from H, hydroxyl, C₁₋₆ alkyl, C₁₋₆ alkoxy or 3- to 6-membered cycloalkyl;
R₅ is selected from C₁₋₆ alkyl or 3- to 6-membered cycloalkyl; and
R₆ is selected from C₁₋₆ alkyl or 3- to 6-membered cycloalkyl, wherein the alkyl and cycloalkyl may be optionally further substituted with one or more R.

Preferably,
R₂ is selected from H, hydroxyl, C₁₋₆ alkyl, C₁₋₆ alkoxy or 3- to 6-membered cycloalkyl;
R₁ and R₄ together with the atoms to which they are attached form 4- to 6-membered cycloalkyl or heterocyclyl fused to a benzene ring, wherein the cycloalkyl and heterocyclyl may be optionally further substituted with one or more R;
R₅ is selected from C₁₋₆ alkyl or 3- to 6-membered cycloalkyl; and
R₆ is selected from C₁₋₆ alkyl or 3- to 6-membered cycloalkyl.

Preferably,
R₂ is independently selected from C₁₋₆ alkyl or 3- to 6-membered cycloalkyl;
R₄ is selected from H;
R₆ is selected from C₁₋₆ alkyl or 3- to 6-membered cycloalkyl, wherein the alkyl and cycloalkyl may be optionally further substituted with one or more R; and
R is selected from F, Cl, Br or I.

Preferably,
R₁ and R₄ together with the atoms to which they are attached form 4- to 6-membered cycloalkyl fused to a benzene ring, wherein the cycloalkyl may be optionally further substituted with one or more R; and
R is selected from C₁₋₆ alkyl or C₁₋₆ alkoxy.

In another preferred embodiment of the present invention, the following compounds are included:

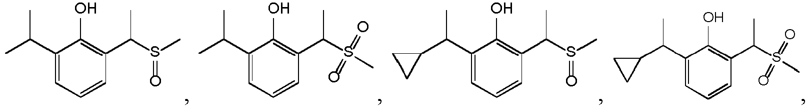

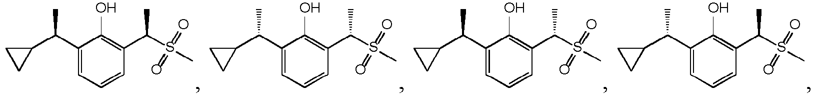

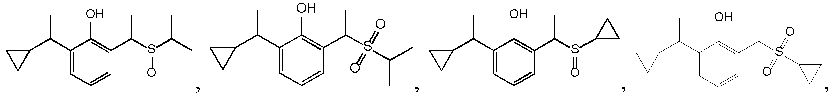

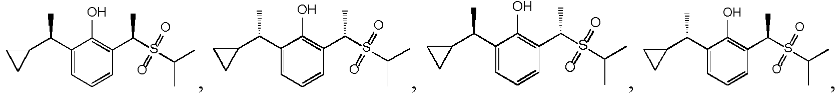

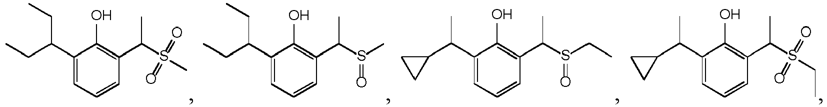

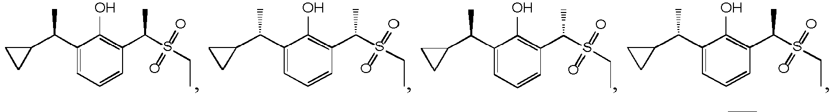

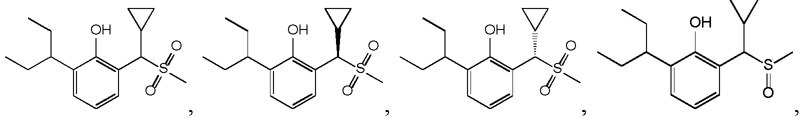

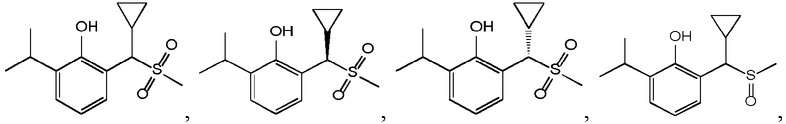

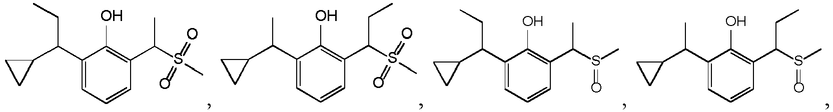

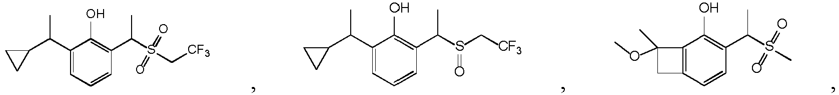

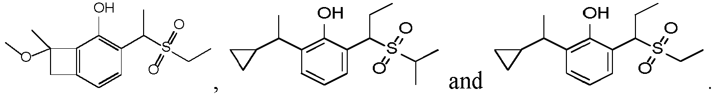

In another aspect, the present invention provides a pharmaceutical composition, comprising the compound or the stereoisomer or pharmaceutically acceptable salt thereof described above in the present invention, and one or more pharmaceutically acceptable carriers.

The pharmaceutical composition involved in the present invention is in any one of the pharmaceutically acceptable dosage forms, such as tablets, capsules, dispersible tablets, granules, injections, lipid emulsions, aerosols, inhalation powders, sprays, oral solutions, and oral suspensions.

The present invention also provides the use of the compound or the stereoisomer or pharmaceutically acceptable salt thereof and the pharmaceutical composition thereof described in the present invention in the preparation of a medicament for inducing and/or maintaining anesthesia in animal or human bodies, facilitating sedation and hypnosis in animal or human bodies, and treating and/or preventing anxiety, depression, insomnia, nausea, vomiting, migraine, schizophrenia, convulsion and epilepsy.

Unless stated to the contrary, the terms used in the description and claims have the following meanings.

"Alkyl" refers to a straight or branched saturated aliphatic hydrocarbon group containing 1 to 20 carbon atoms, preferably alkyl containing 1 to 8 carbon atoms, more preferably alkyl containing 1 to 6 carbon atoms, further preferably alkyl containing 1 to 4 carbon atoms. Examples include methyl, ethyl, n-propyl, isopropyl, etc.

"Alkoxy" refers to -O-alkyl. Examples include methoxy, ethoxy, n-propoxy, isopropoxy, etc.

"Cycloalkoxy" refers to a group formed by bonding the above-mentioned cycloalkyl to an oxygen atom. As 3- to 6-membered cycloalkoxy, examples include cyclopropyloxy, cyclohexyloxy, etc.

"Cycloalkyl" refers to a saturated monocyclic or polycyclic cyclic hydrocarbon substituent, and a cycloalkyl ring comprises 3 to 20 carbon atoms, preferably 3 to 12 carbon atoms, more preferably 3 to 8 carbon atoms, and most preferably 3 to 6 (for example, 3, 4, 5 or 6) carbon atoms. Non-limiting examples of monocyclic cycloalkyl include cyclopropyl, cyclobutyl, cyclopentyl, cyclohexyl, cycloheptyl, cyclooctyl, etc. Examples of polycyclic cycloalkyl include spiro ring, fused ring and bridged ring cycloalkyl.

"Heterocyclyl" refers to a saturated or partially unsaturated monocyclic or polycyclic cyclic hydrocarbon substituent, which comprises 3 to 20 ring atoms, wherein one or more ring atoms are heteroatoms selected from nitrogen, oxygen or S(O)ₘ (wherein m is an integer of 0 to 4), but excluding ring moieties of -O-O-, - O-S- or -S-S-, and the remaining ring atoms are carbon. The cyclic hydrocarbon substituent preferably comprises 3 to 12 ring atoms, of which 1-4 are heteroatoms. The cyclic hydrocarbon substituent preferably comprises 3 to 8 ring atoms, of which 1-3 are heteroatoms. The cyclic hydrocarbon substituent preferably comprises 3 to 6 ring atoms, of which 1-3 are heteroatoms. Non-limiting examples of monocyclic heterocyclyl include azetidinyl, pyrrolidinyl, imidazolidinyl, tetrahydrofuryl, tetrahydropyranyl, tetrahydrothienyl, dihydroimidazolyl, dihydrofuryl, dihydropyrazolyl, dihydropyrrolyl, piperidyl, piperazinyl, morpholinyl, thiomorpholinyl, homopiperazinyl, etc., preferably tetrahydropyranyl, piperazinyl, and pyrrolidinyl. Examples of polycyclic heterocyclyl include spiro ring, fused ring and bridged ring heterocyclyl.

"Stereoisomer" refers to an isomer produced as a result of different spatial arrangements of atoms in molecules, including cis-trans isomers, enantiomers and conformational isomers.

"Optional" or "optionally" or "alternative" means that the events or conditions subsequently described may but not necessarily occur, and the description includes the case where the events or conditions occur and do not occur. For example, "heterocyclyl alternatively substituted with alkyl" means that the alkyl may but not necessarily exist, and the description includes the case where the heterocyclyl is substituted with alkyl and the case where the heterocyclyl is not substituted with alkyl.

### Brief Description of the Drawings

FIG. 1 is a graph showing the changes in heart rate over time after administration of a test compound to Beagle dogs;
FIG. 2 is a graph showing the changes in blood pressure over time after administration of a test compound to Beagle dogs;
FIG. 3 is a graph showing the changes in body temperature over time after administration of a test compound to Beagle dogs.

### Detailed Description of Embodiments

The implementation process and beneficial effects of the present invention are described in detail below by way of specific examples, which are intended to help those skilled in the art better understand the essence and characteristics of the present invention.

### Example 1

### Synthesis of compound 1 (2-isopropyl-6-(1-(methylsulfonyl)ethyl)phenol)

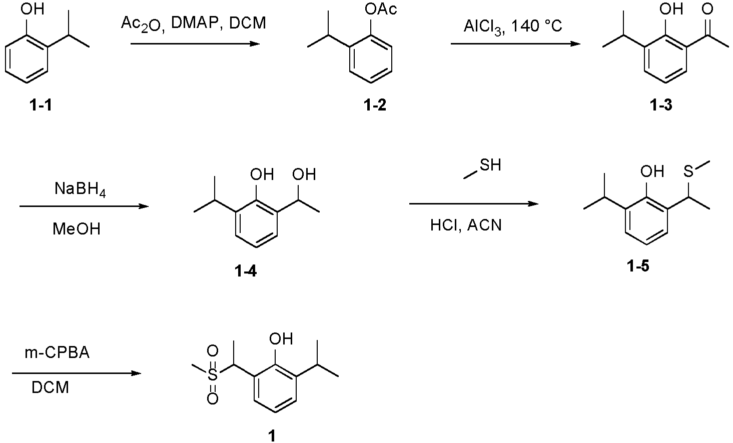

### Step I: Synthesis of compound 1-2 (2-isopropyl phenylacetate)

Compound **1-1** (10 g, 73.43 mmol, 1.0 eq) and DMAP (897 mg, 9.34 mmol) were added to dichloromethane (200 mL). Acetic anhydride (9.74 g, 95.45 mmol, 1.3 eq) was added dropwise at 15°C. After the dropwise addition was completed, the mixture was slowly warmed to room temperature (15°C) and reacted for 16 h. The reaction liquid was neutralized with 1 N hydrochloric acid solution to pH = 6-7, and then extracted with dichloromethane (150 mL × 3). The organic phases were combined and subjected to rotary evaporation to remove the solvent. Column separation was conducted (eluent: V_{Ethyl acetate} : V_{Petroleum ether} = 1 : 10) to obtain compound **1-2** as a yellow transparent oil (**13.16** g, yield: 99.1%).

Characterization data: ¹H NMR (400 MHz, CDCl₃) δ 7.34 - 7.31 (m, 1H), 7.24 - 7.18 (m, 2H), 7.01 - 6.98 (m, 1H), 3.05 - 3.01 (m, 1H), 2.33 (s, 3H), 1.23 (d, *J =* 8.0 Hz, 6H).

### Step II: Synthesis of compound 1-3 (1-(2-hydroxyl-3-isopropylphenyl)ethan-1-one)

Compound **1-2** (13.09 g, 73.45 mmol, 1.0 eq) was mixed with aluminium trichloride (10.58 g, 79.32 mmol, 1.1 eq), and then the reaction mixture was heated to 140°C and reacted for 5 hours. After TLC detection showed that the reaction was completed, the reaction liquid was poured into saturated NH₄Cl (200 mL), and filtration and liquid separation were conducted. The organic phase was subjected to rotary evaporation to remove the solvent, and column separation (eluent : V_{Petroleum ether} : V_{Ethyl acetate} = 50 : 1 to 20 : 1) was conducted to obtain compound **1-3** as a yellow transparent oil (1.61 g, yield: 12.3%).

Characterization data: ¹H NMR (400 MHz, CDCl₃) δ 12.70 (s, 1H), 7.61 - 7.59 (m, 1H), 7.43 - 7.41 (m, 1H), 6.89 - 6.85 (m, 1H), 3.40 - 3.35 (m, 1H), 2.64 (s, 3H), 1.26 - 1.23 (m, 6H).

### Step III: Synthesis of compound 1-4 (2-(1-hydroxyethyl)-6-isopropylphenol)

Compound **1-3** (800 mg, 4.49 mmol, 1.0 eq) was added to dry methanol (15 mL), and then NaBH₄ (255 mg, 6.73 mmol, 1.5 eq) was added. The mixture was reacted at room temperature for 0.5 hours. The reaction liquid was poured into a saturated ammonium chloride aqueous solution (10 mL), and extracted with DCM (50 mL × 4). The organic phases were combined and subjected to rotary evaporation to remove the solvent, so as to obtain compound **1-4** as a yellow oil (809 mg, yield: 100%).

Characterization data: ¹H NMR (400 MHz, CDCl₃) δ 8.13 (s, 1H), 7.15 - 7.13 (m, 1H), 6.85 - 6.81 (m, 2H), 5.10 - 5.04 (m, 1H), 3.41 - 3.32 (m, 1H), 2.40 (s, 1H), 1.62 (d, *J =* 8.0 Hz, 3H), 1.28 - 1.20 (m, 6H).

### Step IV: Synthesis of compound 1-5 (2-isopropyl-6-(1-(methylthio)ethyl)phenol)

Compound **1-4** (0.75 g, 4.16 mmol) was added to methanethiol (2.40 g, 10% propylene glycol solution, 4.99 mmol, 1.2 eq) under nitrogen protection, and hydrochloric acid (230 mg, 6.24 mmol, 1.5 eq) was added dropwise to the reaction liquid. The reaction system was stirred and reacted at 25°C for 16 hours. After TLC (V_{Hexane} : V_{EA} = 5 : 1) detection showed that a majority of raw materials were consumed, the reaction was stopped. The reaction liquid was diluted with H₂O and extracted with ethyl acetate (50 mL × 3). The organic phases were combined, washed with saturated saline and concentrated. The crude product was purified by column chromatography (eluent polarity : V_{n-Hexane} : V_{Ethyl acetate} = 15 : 1 to 12 : 1) to obtain compound **1-5** as a yellow oil (440 mg, yield: 50.3%).

Characterization data: ¹H NMR (400 MHz, CDCl₃) δ 7.38 (s, 1H), 7.17 - 7.15 (m, 1H), 6.92 - 6.90 (m, 1H), 6.85 - 6.82 (m, 1H), 4.07 - 4.02 (m, 1H), 3.40 - 3.32 (m, 1H), 1.94 (d, *J =* 4.0 Hz, 3H), 1.65 (d, *J =* 8.0 Hz, 3H), 1.26 - 1.21 (m, 6H).

### Step V: Synthesis of compound 1 (2-isopropyl-6-(1-(methylsulfonyl)ethyl)phenol)

Under nitrogen protection, compound **1-5** (140 mg, 0.666 mmol) was dissolved in DCM (10 mL), and the system temperature was kept at -5°C - 0°C. Meta-chloroperoxybenzoic acid (240 mg, 1.332 mmol, 2.0 eq) was slowly added to the reaction, and the reaction was conducted at 0°C - 5°C for 1.5 hours. After TLC (V_{Hexane} : V_{EA} = 2 : 1) detection showed that the raw materials were consumed, the reaction was stopped. The reaction liquid was diluted with dichloromethane (20 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 10 : 1) to obtain compound **1** as a yellow oil (60.0 mg, yield: 37.3%).

Characterization data: ¹H NMR (400 MHz, CDCl₃) δ 7.26 - 7.24 (m, 1H), 7.12 - 7.10 (m, 1H), 7.01 - 6.97 (m, 1H), 6.85 (s, 1H), 4.63 - 4.58 (m, 1H), 3.37 - 3.30 (m, 1H), 2.76 (s, 3H), 1.83 (d, *J* = 8.0 Hz, 3H), 1.26 - 1.23 (m, 6H).

### Example 2

### Synthesis of compound 2 (2-isopropyl-6-(1-(methylsulfinyl)ethyl)phenol)

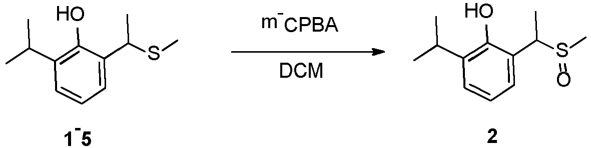

Under nitrogen protection, compound **1-5** (100 mg, 0.475 mmol) was dissolved in DCM (5 mL), and the system temperature was kept at -5°C - 0°C. Meta-chloroperoxybenzoic acid (48.62 mg, 0.237 mmol, 0.5 e.q) was slowly added to the reaction, and the reaction was conducted at 0°C - 5°C for 0.5 hours. After TLC (V_{Hexane} : V_{EA} = 1 : 1) detection showed that the reaction of the raw materials was almost completed, the reaction was stopped. The reaction liquid was diluted with dichloromethane (20 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **2** as a colourless oil (28.0 mg, yield: 26.0%).

Characterization data: ¹H NMR (400 MHz, CDCl₃) δ 9.67 (s, 1H), 7.20 - 7.18 (m, 1H), 6.86 - 6.82 (m, 1H), 6.80 - 6.78 (m, 1H), 3.76 - 3.70 (m, 1H), 3.45 - 3.38 (m, 1H), 2.45 (s, 3H), 1.89 (d, *J* = 8.0 Hz, 3H), 1.24 - 1.21 (m, 6H).

### Example 3 (not encompassed by the wording of the claims)

### Synthesis of compound 3 (2-(1-cyclopropylethyl)-6-(1-(methylthio)ethyl)phenol)

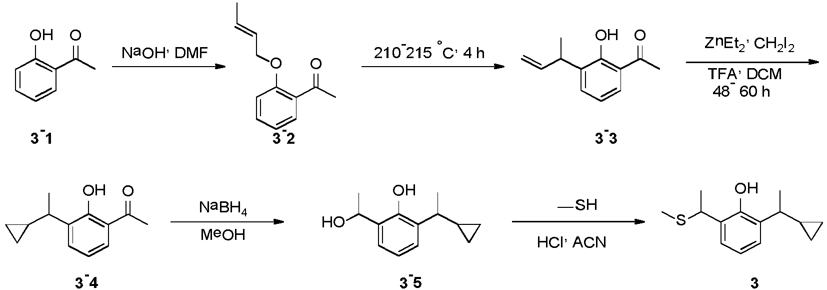

### Step I: Synthesis of compound 3-2 (1-(2-(but-2-en-1-yloxy)phenyl)ethan-1-one)

Compound **3-1** (5.00 g, 73.43 mmol, 1.0 e.q) and NaOH (2.94 g, 73.42 mmol) were added to DMF (80 mL), and the mixture was stirred at 15°C for 30 min. 1-chloro-2-butylene (a cis-trans mixture, 4.32 g, 47.74 mmol, 1.3 e.q) was then slowly added dropwise. After the dropwise addition was completed, the system was reacted at 15°C for additional 16 hours. After TLC detection showed that the reaction was completed, the reaction liquid was slowly poured into ice water (200 mL), and extracted with n-hexane (200 mL × 3). The organic phases were combined, and subjected to rotary evaporation to remove the solvent, so as to obtain compound **3-2** as a yellow transparent oil (6.99 g, yield: 100%).

### Step II: Synthesis of compound 3-3 (1-(3-(but-3-en-2-yl)-2-hydroxyphenyl)ethan-1-one)

Compound **3-2** (6.99 g, 36.72 mmol, 1.0 eq) was added to a 50-mL single-necked flask. Under nitrogen protection, condensation was conducted with air reflux. The reaction mixture was heated to 210°C-215°C and reacted for 3.5 hours. After TLC detection showed that the reaction was completed, the reaction liquid was diluted with ethyl acetate, and subjected to sample stirring and column separation (eluent : V_{Petroleum ether} : V_{Ethyl acetate} = 50 : 1/20 : 1) to obtain compound **3-3** as a yellow transparent oil (1.60 g, yield: 22.9%).

Characterization data: ¹H NMR (400 MHz, CDCl₃) δ 12.70 (s, 1H), 7.63 - 7.61 (m, 1H), 7.39 - 7.36 (m, 1H), 6.88 - 6.85 (m, 1H), 6.05 - 6.00 (m, 1H), 5.11 - 5.05 (m, 2H), 3.98 - 3.97 (m, 1H), 2.64 (s, 3H), 1.34 (d, *J =* 4.0 Hz, 3H).

### Step III: Synthesis of compound 3-4 (1-(3-(1-cyclopropylethyl)-2-hydroxyphenyl)ethan-1-one)

Under nitrogen protection, a 100-mL three-necked flask was subjected to replacement 3-5 times, and DCM (10 mL) was added. The reaction system was cooled to -5°C-0°C, and diethyl zinc (9.46 mL, 2.0 M, 18.92 mmol) was slowly added dropwise to the reaction liquid, with the addition completed in about 10 minutes. Then trifluoroacetic acid (2.16 mg, 18.92 mmol) was added at 5°C-0°C (ice ethanol bath). After about 5 minutes, diiodomethane (6.76 g, 25.23 mmol) was dissolved in DCM (5 mL), and the mixture was added to the reaction liquid using a syringe. The system temperature was kept at -5°C-0°C. After 60 minutes, compound **3-3** (1.2 g, 6.31 mmol) was dissolved in DCM (5 mL), and the mixture was slowly added dropwise to the reaction. After the dropwise addition was completed, the ice bath was removed. The reaction was warmed to 25°C and stirred for additional 48 hours. After ¹HNMR and TLC (V_{Hexane} : V_{EA} = 10 : 1) detection showed that a majority of raw materials were consumed, the reaction was stopped. The reaction liquid was diluted with dichloromethane (20 mL), and washed with a saturated ammonium chloride solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 60 : 1 to 15 : 1) to obtain compound **3-4** as a yellow oil (1.1 g, yield: 85.3%).

Characterization data: ¹H NMR (400 MHz, CDCl₃) δ 12.63 (s, 1H), 7.63 - 7.60 (m, 1H), 7.56 - 7.54 (m, 1H), 6.91 - 6.87 (m, 1H), 2.64 (s, 3H), 2.52 - 2.48 (m, 1H), 1.34 - 1.27 (m, 3H), 1.03 - 0.99 (m, 1H), 0.56 - 0.50 (m, 1H), 0.38 - 0.30 (m, 1H), 0.24 - 0.20 (m, 1H), 0.17 - 0.15 (m, 1H).

### Step IV: Synthesis of compound 3-5 (2-(1-cyclopropylethyl)-6-(1-hydroxyethyl)phenol)

Compound **3-4** (1.1 g, 5.39 mmol, 1.0 eq) was added to dry methanol (15 mL), and then NaBH₄ (265 mg, 7.00 mmol, 1.5 eq) was added in portions. The mixture was reacted at room temperature for 0.5 hours. The reaction liquid was poured into a saturated ammonium chloride aqueous solution (10 mL), and extracted with DCM (50 mL × 2). The organic phases were combined and concentrated to remove the solvent, so as to obtain compound **3-5** as a yellow transparent oil (1.10 g, yield: 99.1%).

Characterization data: ¹H NMR (400 MHz, CDCl₃) δ 8.08 (s, 1H), 7.26 - 7.24 (m, 1H), 7.02 - 6.98 (m, 1H), 6.86 - 6.83 (m, 1H), 5.28 - 5.06 (m, 1H), 2.53 - 2.46 (m, 1H), 1.62 - 1.60 (m, 3H), 1.31 - 1.26 (m, 3H), 1.03 - 0.99 (m, 1H), 0.56 - 0.54 (m, 1H), 0.39 - 0.37 (m, 1H), 0.22 - 0.20 (m, 1H), 0.17 - 0.15 (m, 1H).

### Step V: Synthesis of compound 3 (2-(1-cyclopropylethyl)-6-(1-(methylthio)ethyl)phenol)

Compound **3-5** (1.3 g, 6.30 mmol) was added to acetonitrile (15 mL), and methanethiol (3.94 g, 10% propylene glycol solution, 8.19 mmol, 1.2 e.q.) was added under nitrogen protection. Then hydrochloric acid (344 mg, 9.45 mmol, 1.5 eq) was added dropwise to the reaction liquid. The reaction system was stirred and reacted at 25°C for 4 hours. After TLC (V_{Hexane} : V_{EA} = 5 : 1) detection showed that a majority of raw materials were consumed, the reaction was stopped. The reaction liquid was diluted with H₂O and extracted with ethyl acetate (50 mL × 3). The organic phases were combined, washed with saturated saline and concentrated. The crude product was purified by column chromatography (eluent polarity : V_{n-Hexane} : V_{Ethyl acetate} = 50 : 1 to 20 : 1) to obtain compound **3** as a yellow oil (500 mg, yield: 33.6%).

Characterization data: ¹H NMR (400 MHz, CDCl₃) δ 7.35 - 7.34 (m, 1H), 7.27 - 7.25 (m, 1H), 6.93 - 6.91 (m, 1H), 6.87 - 6.83 (m, 1H), 4.09 - 4.02 (m, 1H), 2.57 - 2.52 (m, 1H), 1.94 (d, *J* = 8.0 Hz, 3H), 1.65 (d, *J* = 8.0 Hz, 3H), 1.30 - 1.24 (m, 3H), 1.04 - 0.95 (m, 1H), 0.53 - 0.50 (m, 1H), 0.35 - 0.30 (m, 1H), 0.20 - 0.15 (m, 2H).

### Example 4

### Synthesis of compound 4 (2-(1-cyclopropylethyl)-6-(1-(methylsulfonyl)ethyl)phenol)

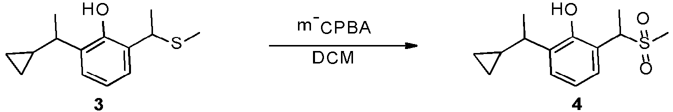

Under nitrogen protection, compound **3** (100 mg, 0.423 mmol) was dissolved in DCM (10 mL), and the system temperature was kept at -5°C - 0°C. Meta-chloroperoxybenzoic acid (165 mg, 0.846 mmol, 2.0 eq) was slowly added to the reaction, and the reaction was conducted at 15°C for 0.5 hours. After TLC (V_{Hexane} : V_{EA} = 1 : 1) detection showed that the raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **4** as a yellow oil (40.0 mg, yield: 35.4%).

Characterization data: ¹H NMR (400 MHz, CDCl₃) δ 7.35 - 7.34 (m, 1H), 7.14 - 7.12 (m, 1H), 7.02 - 6.98 (m, 1H), 6.84 (s, 1H), 4.65 - 4.59 (m, 1H), 2.76 (s, 3H), 2.59 - 2.54 (m, 1H), 1.83 (d, *J =* 8.0 Hz, 3H), 1.30 - 1.25 (m, 3H), 1.06 - 0.99 (m, 1H), 0.60 - 0.54 (m, 1H), 0.46 - 0.40 (m, 1H), 0.26 - 0.13 (m, 2H).

### Example 5

### Synthesis of compound 5 (2-(1-cyclopropylethyl)-6-(1-(methylsulfinyl)ethyl)phenol)

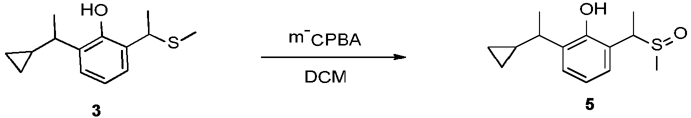

Under nitrogen protection, compound **3** (100 mg, 0.423 mmol) was dissolved in DCM (10 mL), and the system temperature was kept at -5°C - 0°C. Meta-chloroperoxybenzoic acid (77.30 mg, 0.381 mmol, 0.9 eq) was slowly added to the reaction, and the reaction was conducted at 0°C - 5°C for 0.5 hours. After TLC (V_{Hexane} : V_{EA} = 1 : 1) detection showed that a majority of raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **5** as a yellow oil (40.0 mg, yield: 37.7%).

Characterization data: ¹H NMR (400 MHz, CDCl₃) δ 9.61 (s, 1H), 7.32 - 7.30 (m, 1H), 6.88 - 6.79 (m, 2H), 3.77 - 3.71 (m, 1H), 2.59 - 2.56 (m, 1H), 2.45 (s, 3H), 1.88 (d, *J =* 8.0 Hz, 3H), 1.30 - 1.28 (m, 3H), 1.04 - 1.01 (m, 1H), 0.53 - 0.50 (m, 1H), 0.37 - 0.32 (m, 1H), 0.21 - 0.16 (m, 2H).

### Example 6

### Synthesis of compound 6 (2-(1-cyclopropylethyl)-6-(1-(isopropylsulfonyl)ethyl)phenol)

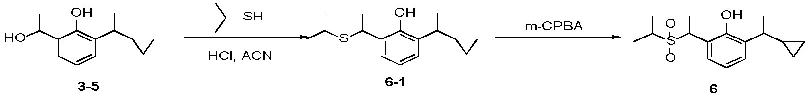

### Step I: Synthesis of compound 6-1 (2-(1-cyclopropylethyl)-6-(1-(isopropylthio)ethyl)phenol)

Compound **3-5** (1.0 g, 4.85 mmol) was added to acetonitrile (10 mL), and isopropylthiol (443.0 mg, 5.82 mmol, 1.2 e.q.) was added under nitrogen protection. Then hydrochloric acid (230 mg, 6.30 mmol, 1.3 e.q.) was added dropwise to the reaction liquid. The reaction system was stirred and reacted at 15°C for 16 hours. After TLC (V_{Hexane} : V_{EA} = 5 : 1) detection showed that a majority of raw materials were consumed, the reaction was stopped. The reaction liquid was diluted with H₂O and extracted with ethyl acetate (50 mL × 3). The organic phases were combined, washed with saturated saline and concentrated. The crude product was purified by column chromatography (eluent polarity : V_{n-Hexane} : V_{Ethyl acetate} = 50 : 1 to 20 : 1) to obtain compound **6-1** as a yellow oil (600 mg, yield: 46.8%).

Characterization data: ¹H NMR (400 MHz, CDCl₃) δ 7.70 (d, *J* = 8.0 Hz, 1H), 7.26 - 7.24 (m, 1H), 6.92 - 6.90 (m, 1H), 6.86 - 6.82 (m, 1H), 4.20 - 4.15 (m, 1H), 2.70 - 2.63 (m, 1H), 2.58 - 2.51 (m, 1H), 1.63 (d, *J* = 8.0 Hz, 3H), 1.30 - 1.24 (m, 3H), 1.17 - 1.14 (m, 6H), 1.04 - 0.99 (m, 1H), 0.56 - 0.52 (m, 1H), 0.39 - 0.34 (m, 1H), 0.22 - 0.15 (m, 2H).

### Step II: Synthesis of compound 6 (2-(1-cyclopropylethyl)-6-(1-(isopropylsulfonyl)ethyl)phenol)

Under nitrogen protection, compound **6-1** (100 mg, 0.378 mmol) was dissolved in DCM (10 mL), and the system temperature was kept at -5°C-0°C. Meta-chloroperoxybenzoic acid (154 mg, 85%, 0.756 mmol, 2.0 eq) was slowly added to the reaction, and the reaction was conducted at 15°C for 0.5 hours. After TLC (V_{Hexane} : V_{EA} = 1 : 1) detection showed that the raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **6** as a yellow oil (50.0 mg, yield: 44.6%).

Characterization data: ¹H NMR (400 MHz, CDCl₃) δ 7.35 - 7.31 (m, 1H), 7.10 - 7.08 (m, 1H), 6.97 - 6.93 (m, 1H), 4.60 - 4.58 (m, 1H), 3.18 - 3.10 (m, 1H), 2.60 - 2.54 (m, 1H), 1.81 (d, *J* = 8.0 Hz, 3H), 1.31 - 1.27 (m, 6H), 1.26 - 1.17 (m, 3H), 1.04 - 0.98 (m, 1H), 0.56 - 0.52 (m, 1H), 0.37 - 0.34 (m, 1H), 0.22 - 0.15 (m, 2H).

### Example 7

### Synthesis of compound 7 (2-(1-cyclopropylethyl)-6-(1-(isopropylsulfinyl)ethyl)phenol)

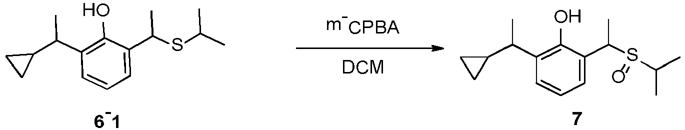

Under nitrogen protection, compound **6-1** (100 mg, 0.378 mmol) was dissolved in DCM (10 mL), and the system temperature was kept at -5°C-0°C. Meta-chloroperoxybenzoic acid (69.1 mg, 0.341 mmol, 0.9 eq) was slowly added to the reaction, and the reaction was conducted at 0°C-5°C for 0.5 hours. After TLC (V_{Hexane} : V_{EA} = 1 : 1) detection showed that a majority of raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **7** as a yellow oil (30.0 mg, yield: 28.3%).

Characterization data: ¹H NMR (400 MHz, CDCl₃) δ 7.35 - 7.31 (m, 1H), 7.10 - 7.08 (m, 1H), 6.97 - 6.93 (m, 1H), 4.60 - 4.58 (m, 1H), 2.68 - 2.63 (m, 1H), 2.45 - 2.38 (m, 1H), 1.81 (d, *J* = 8.0 Hz, 3H), 1.31 - 1.27 (m, 6H), 1.26 - 1.17 (m, 3H), 1.04 - 0.98 (m, 1H), 0.56 - 0.54 (m, 1H), 0.39 - 0.32 (m, 1H), 0.22 - 0.17 (m, 2H).

### Example 8

### Synthesis of compound 8 (2-(1-cyclopropylethyl)-6-(1-(cyclopropylsulfonyl)ethyl)phenol)

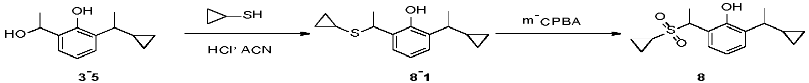

### Step I: Synthesis of compound 8-1 (2-(1-cyclopropylethyl)-6-(1-(cyclopropylthio)ethyl)phenol)

Compound **3-5** (1.0 g, 4.85 mmol) was added to acetonitrile (10 mL), and cyclopropylthiol (431.2 mg, 5.82 mmol, 1.2 eq) was added under nitrogen protection. Then concentrated hydrochloric acid (230 mg, 6.30 mmol, 1.3 eq) was added dropwise to the reaction liquid. The reaction system was stirred and reacted at 15°C for 12 hours. After TLC (V_{Hexane} : V_{EA} = 5 : 1) detection showed that a majority of raw materials were consumed, the reaction was stopped. The reaction liquid was diluted with H₂O and extracted with ethyl acetate (50 mL × 3). The organic phases were combined, washed with saturated saline and concentrated. The crude product was purified by column chromatography (eluent polarity : V_{n-Hexane} : V_{Ethyl acetate} = 50 : 1 to 20 : 1) to obtain compound **8-1** as a yellow oil (490 mg, yield: 38.5%).

Characterization data: ¹H NMR (400 MHz, CDCl₃) δ 7.35 - 7.34 (m, 1H), 7.27 - 7.25 (m, 1H), 6.93 - 6.91 (m, 1H), 6.87 - 6.83 (m, 1H), 4.09 - 4.02 (m, 1H), 2.57 - 2.52 (m, 1H), 2.05 - 2.00 (m, 1H), 1.94 (d, *J* = 8.0 Hz, 3H), 1.65 (d, *J* = 8.0 Hz, 3H), 1.04 - 0.95 (m, 2H), 0.74 - 0.58 (m, 2H), 0.59 - 0.50 (m, 1H), 0.39 - 0.30 (m, 2H), 0.24 - 0.12 (m, 2H).

### Step II: Synthesis of compound 8 (2-(1-cyclopropylethyl)-6-(1-(cyclopropylsulfonyl)ethyl)phenol)

Under nitrogen protection, compound **8-1** (100 mg, 0.381 mmol) was dissolved in DCM (10 mL), and the system temperature was kept at -5°C-0°C. Meta-chloroperoxybenzoic acid (155 mg, 0.762 mmol, 2.0 eq) was slowly added to the reaction, and the reaction was conducted at 15°C for 0.5 hours. After TLC (V_{Hexane} : V_{EA} = 1 : 1) detection showed that the raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **8** as a yellow solid (60.0 mg, yield: 53.4%).

Characterization data: ¹H NMR (400 MHz, CDCl₃) δ 7.35 - 7.34 (m, 1H), 7.14 - 7.12 (m, 1H), 7.02 - 6.98 (m, 1H), 6.84 (s, 1H), 4.65 - 4.59 (m, 1H), 2.59 - 2.54 (m, 1H), 2.45 - 2.35 (m, 1H), 1.83 (d, *J* = 8.0 Hz, 3H), 1.30 - 1.24 (m, 3H), 1.04 - 0.95 (m, 1H), 0.83 - 0.80 (m, 1H), 0.65 - 0.60 (m, 2H), 0.53 - 0.50 (m, 1H), 0.45 - 0.40 (m, 1H), 0.35 - 0.30 (m, 1H), 0.20 - 0.15 (m, 2H).

### Example 9

### Synthesis of compound 9 (2-(1-cyclopropylethyl)-6-(1-(cyclopropylsulfinyl)ethyl)phenol)

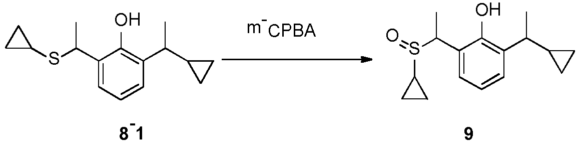

Under nitrogen protection, compound **8-1** (100 mg, 0.381 mmol) was dissolved in DCM (10 mL), and the system temperature was kept at -5°C-0°C. Meta-chloroperoxybenzoic acid (69.6 mg, 0.343 mmol, 0.9 eq) was slowly added to the reaction, and the reaction was conducted at 0°C-5°C for 0.5 hours. After TLC (V_{Hexane} : V_{EA} = 1 : 1) detection showed that a majority of raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **9** as a yellow transparent oil (38.0 mg, yield: 35.8%).

Characterization data: ¹H NMR (400 MHz, CDCl₃) δ 9.63 (s, 1H), 7.35 - 7.32 (m, 1H), 6.89 - 6.75 (m, 2H), 3.77 - 3.71 (m, 1H), 2.59 - 2.56 (m, 1H), 2.45 - 2.34 (m, 1H), 1.88 (d, *J =* 8.0 Hz, 3H), 1.30 - 1.24 (m, 3H), 1.04 - 0.95 (m, 1H), 0.83 - 0.80 (m, 1H), 0.65 - 0.60 (m, 2H), 0.53 - 0.50 (m, 1H), 0.45 - 0.40 (m, 1H), 0.35 - 0.30 (m, 1H), 0.20 - 0.15 (m, 2H).

### Example 10 (not encompassed by the wording of the claims)

### Synthesis of compound 10 (1-(2-hydroxyl-3-isopropylphenyl)ethyl acetate)

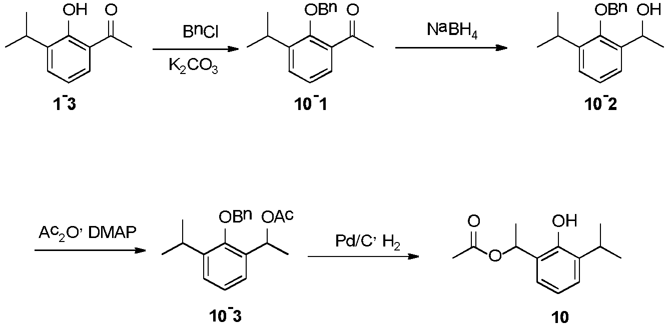

### Step I: Synthesis of compound 10-1 (1-(2-(benzyloxy)-3-isopropylphenyl)ethan-1-one)

Compound **1-3** (2.00 g, 11.22 mmol, 1.0 eq) was added to a 100-mL single-necked flask, dry methanol (40 mL) and potassium carbonate (2.33 g, 16.83 mmol, 1.5 eq) were added, and then benzyl chloride (1.85 g, 14.59 mmol, 1.3 eq) was added. The reaction mixture was heated to 30°C and reacted for 2 h under nitrogen protection. After TLC detection showed that the reaction was completed, the reaction liquid was diluted with ethyl acetate, and subjected to sample stirring and column separation (eluent : V_{Petroleum ether} : V_{Ethyl acetate} = 100 : 1 to 50 : 1) to obtain compound **10-1** as a yellow oil (2.5 g, yield: 83.3%).

Characterization data: ¹H NMR (400 MHz, CDCl₃) δ 7.66 - 7.64 (m, 1H), 7.47 -7.43 (m, 2H), 7.37 - 7.35 (m, 3H), 6.88 - 6.84 (m, 2H), 5.06 (s, 2H), 2.46 - 2.42 (m, 1H), 2.26 (s, 3H), 1.34 - 1.21 (m, 6H).

### Step II: Synthesis of compound 10-2 (1-(2-(benzyloxy)-3-isopropylphenyl)ethan-1-ol)

Compound **10-1** (2.0 g, 7.45 mmol, 1.0 eq) was added to a 100-mL single-necked flask, and dry methanol (30 mL) and sodium borohydride (0.31 g, 8.20 mmol, 1.1 e.q) were added. The reaction mixture was reacted at 15°C for 0.5 hours. After TLC detection showed that the reaction was completed, the reaction liquid was extracted with DCM (100 mL × 2), and purified by column chromatography (eluent polarity : V_{Petroleum ether} : V_{Ethyl acetate} = 15 : 1 to 5 : 1) to obtain compound **10-2** as a yellow oil (2.00 g, yield: 100.0%).

Characterization data: ¹H NMR (400 MHz, CDCl₃) δ 7.65 - 7.61 (m, 1H), 7.48 - 7.44 (m, 2H), 7.39 - 7.35 (m, 3H), 6.88 - 6.84 (m, 2H), 5.08 (s, 2H), 3.65 (s, 1H), 2.46 - 2.42 (m, 2H), 1.91 (d, *J* = 8.0 Hz, 3H), 1.34 - 1.21 (m, 6H).

### Step III: Synthesis of compound 10-3 (1-(2-(benzyloxy)-3-isopropylphenyl)ethyl acetate)

Compound **10-2** (1.5 g, 5.55 mmol, 1.0 e.q) was added to a 100-mL single-necked flask, dry DCM (30 mL) and DMAP (0.678 g, 5.55 mmol, 1.0 e.q) were added, and then acetic anhydride (736.3 mg, 7.21 mmol, 1.3 e.q) was added. The reaction mixture was reacted at 15°C for 0.5 hours. After TLC detection showed that the reaction was completed, the reaction mixture was extracted with DCM (100 mL × 2). The reaction liquid was washed with hydrochloric acid (1 N), extracted, subjected to liquid separation and dried to obtain compound **10-3** as a yellow transparent oil (1.50 g, yield: 86.7%).

Characterization data: ¹H NMR (400 MHz, CDCl₃) δ 7.65 - 7.61 (m, 1H), 7.48 - 7.44 (m, 2H), 7.39 - 7.35 (m, 3H), 6.88 - 6.84 (m, 2H), 6.05 - 6.00 (m, 1H), 5.08 (s, 2H), 2.46 - 2.42 (m, 1H), 2.12 (s, 3H), 1.93 (d, *J* = 8.0 Hz, 3H), 1.34 - 1.21 (m, 6H).

### Step IV: Synthesis of compound 10 (1-(2-hydroxyl-3-isopropylphenyl)ethyl acetate)

Under the protection of a hydrogen balloon 103.42 kPa (15 psi), compound **10-3** (300 mg, 0.96 mmol, 1.0 eq) was added to dry THF (10 mL), and palladium on carbon (200 g, pd: 10%) was added. The reaction mixture was reacted at 15°C in the hydrogen balloon 103.42 kPa (15 psi) for 5 hours. After TLC detection showed that the reaction was completed, the reaction compound was filtered through celite, washed, dried and concentrated to obtain a product, which was purified by column chromatography to obtain compound **10** as a light yellow oil (100 mg, yield: 46.9%).

¹H NMR (400 MHz, CDCl₃) δ 7.64 (s, 1H), 7.19 - 7.18 (m, 2H), 6.94 - 6.90 (m, 1H), 6.05 - 6.00 (m, 1H), 3.40 - 3.33 (m, 1H), 2.08 (s, 3H), 1.65 (d, *J =* 8.0 Hz, 3H), 1.24 - 1.21 (m, 6H).

### Example 11

### Synthesis of compound 11 (2-(1-cyclopropylethyl)-6-(1-(ethylsulfonyl)ethyl)phenol)

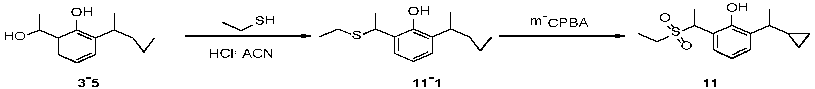

### Step I: Synthesis of compound 11-1 (2-(1-cyclopropylethyl)-6-(1-(ethylthio)ethyl)phenol)

Compound **3-5** (1.0 g, 4.85 mmol) was added to ethanethiol (361 mg, 5.81 mmol, 1.2 eq) under nitrogen protection, and hydrochloric acid (229 mg, 6.30 mmol, 1.3 eq) was added dropwise to the reaction liquid. The reaction system was stirred and reacted at 15°C for 10 hours. After TLC (V_{Hexane} : V_{EA} = 5 : 1) detection showed that a majority of raw materials were consumed, the reaction was stopped. The reaction liquid was diluted with H₂O and extracted with ethyl acetate (50 mL × 3). The organic phases were combined, washed with saturated saline and concentrated. The crude product was purified by column chromatography (eluent polarity : V_{n-Hexane} : V_{Ethyl acetate} = 50 : 1 to 20 : 1) to obtain compound **11-1** as a yellow oil (700 mg, yield: 57.7%).

Characterization data: ¹H NMR (400 MHz, CDCl₃) δ 7.50 (d, *J* = 8.0 Hz, 1H), 7.27 - 7.25 (m, 1H), 6.93 - 6.91 (m, 1H), 6.86 - 6.83 (m, 1H), 4.18 - 4.12 (m, 1H), 2.58 - 2.52 (m, 1H), 2.40 - 2.34 (m, 2H), 1.64 (d, *J* = 8.0 Hz, 3H), 1.31 - 1.28 (m, 3H), 1.19 - 1.15 (m, 3H), 1.05 - 0.97 (m, 1H), 0.55 - 0.54 (m, 1H), 0.35 - 0.30 (m, 1H), 0.20 - 0.16 (m, 2H).

### Step II: Synthesis of compound 11 (2-(1-cyclopropylethyl)-6-(1-(ethylsulfonyl)ethyl)phenol)

Under nitrogen protection, compound **11-1** (200 mg, 0.798 mmol) was dissolved in DCM (10 mL), and the system temperature was kept at -5°C - 0°C. Meta-chloroperoxybenzoic acid (325 mg, 1.60 mmol, 2.0 eq) was slowly added to the reaction, and the reaction was conducted at 15°C for 0.5 hours. After TLC (V_{Hexane} : V_{EA} = 1 : 1) detection showed that the raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **11** as a light yellow oil (180.0 mg, yield: 82%).

Characterization data: ¹H NMR (400 MHz, CDCl₃) δ 7.38 - 7.33 (m, 1H), 7.12 - 7.10 (m, 2H), 6.99 - 6.96 (m, 1H), 4.59 - 4.55 (m, 1H), 2.94 - 2.88 (m, 2H), 2.58 - 2.54 (m, 1H), 1.83 (d, *J* = 8.0 Hz, 3H), 1.34 - 1.32 (m, 3H), 1.30 - 1.27 (m, 3H), 1.05 - 1.01 (m, 1H), 0.58 - 0.56 (m, 1H), 0.42 - 0.40 (m, 1H), 0.23 - 0.15 (m, 2H).

### Example 12

### Synthesis of compound 12 (2-(1-cyclopropylethyl)-6-(1-(ethylsulfinyl)ethyl)phenol)

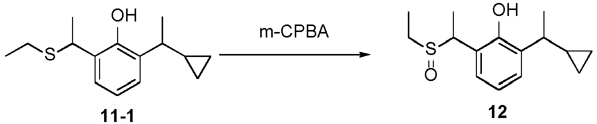

Under nitrogen protection, compound **11-1** (200 mg, 0.798 mmol) was dissolved in DCM (10 mL), and the system temperature was kept at -5°C - 0°C. Meta-chloroperoxybenzoic acid (146 mg, 0.718 mmol, 0.9 eq) was slowly added to the reaction, and the reaction was conducted at 0°C - 5°C for 0.5 hours. After TLC (V_{Hexane} : V_{EA} = 1 : 1) detection showed that a majority of raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **12** as a yellow oil (40.0 mg, yield: 18.8%).

Characterization data: ¹H NMR (400 MHz, CDCl₃) δ 9.79 (s, 1H), 7.32 - 7.29 (m, 1H), 6.86 - 6.79 (m, 2H), 3.82 - 3.80 (m, 1H), 2.64 - 2.56 (m, 2H), 2.40 -2.34 (m, 1H), 1.83 (d, *J =* 8.0 Hz, 3H), 1.34 - 1.29 (m, 3H), 1.24 - 1.19 (m, 3H), 1.05 - 1.00 (m, 1H), 0.54 - 0.51 (m, 1H), 0.38 - 0.35 (m, 1H), 0.21 - 0.16 (m, 2H).

### Example 13 (not encompassed by the wording of the claims)

### Synthesis of compound 13 (2-(1-cyclopropylethyl)-6-(1-(methylthio)propyl)phenol)

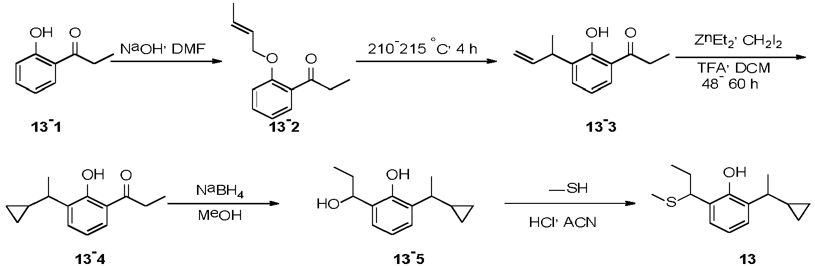

### Step I: Synthesis of compound 13-2 (1-(2-(but-2-en-1-yloxy)phenyl)propan-1-one)

Compound **13-1** (10.0 g, 66.59 mmol, 1.0 eq) and NaOH (4.0 g, 99.88 mmol, 1.5 eq) were added to anhydrous DMF (150 mL), and the mixture was stirred at 15°C for 30 minutes. Chlorobutene (a cis-trans mixture, 7.24 g, 79.91 mmol, 1.3 eq) was then added dropwise at 15°C. After the dropwise addition was completed, the mixture was slowly warmed to room temperature (15°C) and reacted for 16 hours. The reaction liquid was slowly poured into ice water (500 mL), and extracted with n-hexane (400 mL × 3). The organic phases were combined, and subjected to rotary evaporation to remove the solvent. The crude product was subjected to sample stirring, and purified by column chromatography (eluent polarity : V_{n-Hexane} : V_{Ethyl acetate} = 200 : 1) to obtain compound **13-2** as a yellow transparent oil (7.7 g, yield: 56.6%).

Characterization data: ¹H NMR (400 MHz, CDCl₃) δ 7.70 - 7.66 (m, 1H), 7.43 - 7.41 (m, 1H), 7.00 - 6.93 (m, 2H), 5.87 - 5.71 (m, 2H), 4.68 - 4.54 (m, 2H), 3.04 - 2.98 (m, 2H), 1.78 (d, *J =* 8.0 Hz, 3H), 1.18 - 1.16 (m, 3H).

### Step II: Synthesis of compound 13-3 (1-(3-(but-3-en-2-yl)-2-hydroxyphenyl)propan-1-one)

Compound **13-2** (7.70 g, 37.72 mmol, 1.0 eq) was added to a 50-mL single-necked flask. Under nitrogen protection, condensation was conducted with air reflux. The reaction mixture was heated to 210°C-215°C and reacted for 4.0 hours. After TLC detection showed that the reaction was completed, the reaction liquid was diluted with ethyl acetate, and subjected to sample stirring and column separation (eluent : V_{Petroleum ether} : V_{Ethyl acetate} = 100 : 1 to 50 : 1) to obtain compound **13-3** as a yellow oil (6.80 g, yield: 88.3%).

Characterization data: ¹H NMR (400 MHz, CDCl₃) δ 12.81 (s, 1H), 7.66 - 7.64 (m, 1H), 7.37 - 7.35 (m, 1H), 6.88 - 6.84 (m, 1H), 6.05 - 6.01 (m, 1H), 5.08 - 5.06 (m, 2H), 4.01 - 3.97 (m, 1H), 3.06 - 3.02 (m, 2H), 1.34 (d, *J* = 4.0 Hz, 3H), 1.24 - 1.20 (m, 3H).

### Step III: Synthesis of compound 13-4 (1-(3-(1-cyclopropylethyl)-2-hydroxyphenyl)propan-1-one)

Under nitrogen protection, a 100-mL three-necked flask was subjected to replacement 3-5 times, and DCM (20 mL) was added. The reaction system was cooled to -5°C-0°C, and diethyl zinc (14.69 mL, 2.0 M, 29.37 mmol) was slowly added dropwise to the reaction liquid, with the addition completed in about 10 minutes. Then trifluoroacetic acid (3.35 g, 29.37 mmol) was added in an ice ethanol bath. After about 5 minutes, diiodomethane (10.49 g, 39.16 mmol) was dissolved in DCM (5 mL), and the mixture was added to the reaction liquid using a syringe. The system temperature was kept at -5°C-0°C. After 60 minutes, compound 13-3 (2.00 g, 9.79 mmol) was dissolved in DCM (5 mL), and the mixture was slowly added dropwise to the reaction. After the dropwise addition was completed, the ice bath was removed. The reaction was stirred at -5°C-0°C for 3 hours, and then warmed to 15°C and stirred for additional 48 hours. After ¹H NMR and TLC (V_{Hexane} : V_{EA} = 10 : 1) detection showed that a majority of raw materials were consumed, the reaction was stopped. The reaction liquid was diluted with dichloromethane (200 mL), and washed with a saturated ammonium chloride solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 100 : 1 to 50 : 1) to obtain compound **13-4** as a yellow oil (1.80 g, yield: 84.1%).

Characterization data: ¹H NMR (400 MHz, CDCl₃) δ 12.73 (s, 1H), 7.66 - 7.63 (m, 1H), 7.55 - 7.53 (m, 1H), 6.90 (t, *J* = 8.0 Hz, 1H), 3.08 - 3.03 (m, 2H), 2.52 - 2.48 (m, 1H), 1.31 (d, *J* = 4.0 Hz, 3H), 1.24 - 1.20 (m, 3H), 1.03 - 0.97 (m, 1H), 0.59 - 0.53 (m, 1H), 0.41 - 0.35 (m, 1H), 0.26 - 0.20 (m, 1H), 0.16 - 0.12 (m, 1H).

### Step IV: Synthesis of compound 13-5 (2-(1-cyclopropylethyl)-6-(1-hydroxypropyl)phenol)

Compound **13-4** (3.30 g, 15.14 mmol, 1.0 eq) was added to dry methanol (50 mL), and then NaBH₄ (624 mg, 16.49 mmol, 1.1 eq) was added. The mixture was reacted at room temperature for 0.5 hours. The reaction liquid was poured into a saturated ammonium chloride aqueous solution (10 mL), and extracted with DCM (200 mL × 3). The organic phases were combined, washed with saturated saline, and dried over anhydrous sodium sulphate. The crude product was purified by column chromatography (eluent polarity : V_{PE} : V_{EA} = 100 : 1 to 20 : 1) to obtain compound **13-5** as a yellow oil (2.00 g, yield: 60.1%).

Characterization data: ¹H NMR (400 MHz, CDCl₃) δ 8.10 (s, 1H), 7.25 - 7.23 (m, 1H), 6.82 - 6.79 (m, 2H), 4.75 (t, *J* = 8.0 Hz, 1H), 2.52 - 2.45 (m, 2H), 1.98 - 1.84 (m, 2H), 1.30 (dd, *J* = 8.0 Hz, 2.0 Hz, 3H), 1.05- 1.01 (m, 1H), 0.98 (t, *J* = 8.0 Hz, 3H), 0.56 - 0.52 (m, 1H), 0.41 - 0.35 (m, 1H), 0.22 - 0.14 (m, 2H).

### Step V: Synthesis of compound 13 (2-(1-cyclopropylethyl)-6-(1-(methylthio)propyl)phenol)

Compound **13-5** (400 mg, 1.82 mmol) was added to acetonitrile (10 mL), and methanethiol (1.05 g, 10% propylene glycol solution, 2.18 mmol, 1.2 eq) was added under nitrogen protection. Then hydrochloric acid (72.82 mg, 2.00 mmol, 1.1 eq) was added dropwise to the reaction liquid. The reaction system was stirred and reacted at 25°C for 6 hours. After TLC (V_{Hexane} : V_{EA} = 5 : 1) detection showed that a majority of raw materials were consumed, the reaction was stopped. The reaction liquid was diluted with water and extracted with ethyl acetate (30 mL × 3). The organic phases were combined, washed with saturated saline and concentrated. The crude product was purified by column chromatography (eluent polarity : V_{n-Hexane} : V_{Ethyl acetate} = 1 : 0 to 100 : 1) to obtain compound **13** as a yellow oil (110 mg, yield: 24.2%).

Characterization data: ¹H NMR (400 MHz, CDCl₃) δ 7.43 (d, *J* = 4.0 Hz, 1H), 7.26 - 7.25 (m, 1H), 6.87 - 6.81 (m, 2H), 3.80 (t, *J* = 8.0 Hz, 1H), 2.58 - 2.53 (m, 1H), 2.00 - 1.92 (m, 2H), 1.88 (d, *J* = 2.0 Hz, 3H), 1.30 (dd, *J* = 8.0 Hz, 4.0 Hz, 3H), 1.03 - 1.00 (m, 1H), 0.98 (t, *J* = 8.0 Hz, 3H), 0.54 - 0.52 (m, 1H), 0.38 - 0.35 (m, 1H), 0.20 - 0.14 (m, 2H).

### Example 14

### Synthesis of compound 14 (2-(1-cyclopropylethyl)-6-(1-(methylsulfonyl)propyl)phenol)

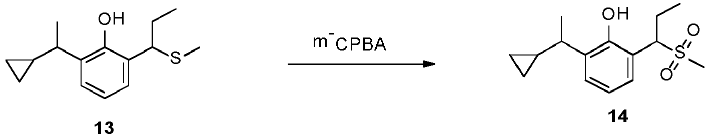

Under nitrogen protection, compound **13** (100 mg, 0.439 mmol) was dissolved in DCM (10 mL), and the system temperature was kept at -5°C-0°C. Meta-chloroperoxybenzoic acid (164 mg, 0.878 mmol, 2.0 eq) was slowly added to the reaction, and the reaction was conducted at 15°C for 0.5 hours. After TLC (V_{Hexane} : V_{EA} = 2 : 1) detection showed that the raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 10 : 1) to obtain compound **14** as a yellow oil (85.0 mg, yield: 75.2%).

Characterization data: ¹H NMR (400 MHz, CDCl₃) δ 7.35 - 7.32 (m, 1H), 7.10 - 7.05 (m, 1H), 7.00 - 6.96 (m, 1H), 4.48 - 4.28 (m, 1H), 2.73 (s, 3H), 2.62 - 2.54 (m, 1H), 2.48 - 2.42 (m, 1H), 2.32 - 2.18 (m, 1H), 1.30 (dd, *J* = 8.0, 4.0 Hz, 3H), 1.04 - 0.99 (m, 1H), 0.95 (t, *J* = 8.0 Hz, 3H), 0.59 - 0.54 (m, 1H), 0.44 - 0.42 (m, 1H), 0.24 - 0.12 (m, 2H).

### Example 15

### Synthesis of compound 15 (2-(1-cyclopropylethyl)-6-(1-(isopropylsulfonyl)propyl)phenol)

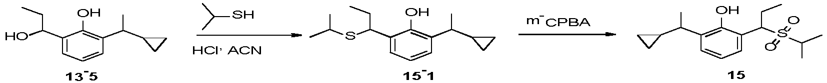

### Step I: Synthesis of compound 15-1 (2-(1-cyclopropylethyl)-6-(1-(isopropylthio)propyl)phenol)

Compound **13-5** (200 mg, 0.91 mmol) was added to acetonitrile (5 mL), and isopropylthiol (82.97 mg, 1.09 mmol, 1.2 eq) was added under nitrogen protection. Then hydrochloric acid (36.41 mg, 0.998 mmol, 1.1 eq) was added dropwise to the reaction liquid. The reaction system was stirred and reacted at 25°C for 6 hours. After TLC (V_{Hexane} : V_{EA} = 10 : 1) detection showed that a majority of raw materials were consumed, the reaction was stopped. The reaction liquid was diluted with H₂O and extracted with ethyl acetate (20 mL × 3). The organic phases were combined, washed with saturated saline and concentrated. The crude product was purified by column chromatography (eluent polarity : V_{n-Hexane} : V_{Ethyl acetate} = 1 : 0 to 100 : 1) to obtain compound **15-1** as a yellow oil (250 mg, yield: 98.8%).

Characterization data: ¹H NMR (400 MHz, CDCl₃) δ 7.78 (d, *J* = 8.0 Hz, 1H), 7.26 -7.24 (m, 1H), 6.85 - 6.79 (m, 2H), 3.91 (q, *J* = 12.0, 8.0, 4.0 Hz, 1H), 2.62 - 2.52 (m, 2H), 1.99 - 1.84 (m, 2H), 1.30 - 1.27 (m, 3H), 1.22 (dd, *J* = 8.0, 4.0 Hz, 3H), 1.16 (dd, *J* = 8.0, 4.0 Hz, 3H), 1.05 - 0.98 (m, 1H), 0.96 (dt, *J* = 8.0 Hz, 3H), 0.56 - 0.49 (m, 1H), 0.39 - 0.31 (m, 1H), 0.21 - 0.13 (m, 2H).

### Step II: Synthesis of compound 15 (2-(1-cyclopropylethyl)-6-(1-(isopropylsulfonyl)propyl)phenol)

Under nitrogen protection, compound **15-1** (200 mg, 0.719 mmol) was dissolved in DCM (10 mL), and the system temperature was kept at -5°C-0°C. Meta-chloroperoxybenzoic acid (291 mg, 1.438 mmol, 2.0 eq) was slowly added to the reaction, and the reaction was conducted at 15°C for 0.5 hours. After TLC (V_{Hexane} : V_{EA} = 3 : 1) detection showed that the raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 15 : 1) to obtain compound **15** as a white solid (160 mg, yield: 71.7%).

Characterization data: ¹H NMR (400 MHz, CDCl₃) δ 8.08 (d, J = 8.0 Hz, 1H), 7.33 - 7.31 (m, 1H), 7.00 - 6.79 (m, 2H), 4.68 - 4.52 (m, 1H), 3.12 - 3.06 (m, 1H), 2.63 - 2.55 (m, 1H), 2.46 - 2.29 (m, 2H), 1.34 - 1.24 (m, 9H), 1.02 - 1.00 (m, 1H), 0.89 (t, J = 8.0 Hz, 3H), 0.57 - 0.53 (m, 1H), 0.42 - 0.35 (m, 1H), 0.23 - 0.12 (m, 2H).

### Example 16

### Synthesis of compound 16 (2-(cyclopropyl(methylsulfonyl)methyl)-6-isopropylphenol)

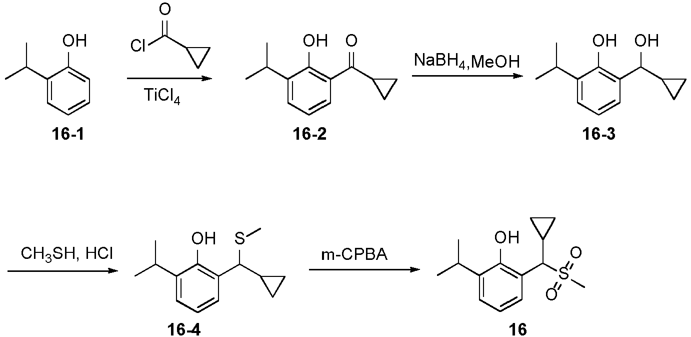

### Step I: Synthesis of compound 16-2 (cyclopropyl(2-hydroxyl-3-isopropylphenyl)methanone)

Compound **16-1** (4.0 g, 29.37 mmol, 1.0 eq) was added to a 100-mL single-necked flask, dry DCM (60 mL) and cyclopropanecarbonyl chloride (3.68 g, 35.24 mmol, 1.2 eq) were added, and then titanium tetrachloride (6.69 g, 35.24 mmol, 1.2 e.q) was added slowly to the reaction. The reaction mixture was kept at -30°C and reacted for 2 hours under nitrogen protection. After TLC detection showed that the reaction was completed, the reaction liquid was diluted with ethyl acetate, and subjected to sample stirring and column separation (eluent : V_{Petroleum ether} : V_{Ethyl acetate} = 100 : 1 to 50 : 1) to obtain compound **16-2** as a yellow oil (200 mg, yield: 3.3%).

Characterization data: ¹H NMR (400 MHz, CDCl₃) δ 12.96 (s, 1H), 7.86 (d, *J* = 8.0 Hz, 1H), 7.45 (d, *J* = 8.0 Hz, 1H), 6.93 (t, *J* = 6.0 Hz, 1H), 3.41 - 3.32 (m, 1H), 2.75 - 2.68 (m, 1H), 1.32 - 1.27 (m, 1H), 1.26 (d, *J* = 6.0 Hz, 6H), 1.11 - 1.06 (m, 1H), 1.05 -0.99 (m, 2H).

### Step II: Synthesis of compound 16-3 (2-(cyclopropyl(hydroxyl)methyl)-6-isopropylphenol)

Compound **16-2** (200 mg, 0.979 mmol, 1.0 eq) was added to a 100-mL single-necked flask, and dry methanol (8 mL) and sodium borohydride (40.75 mg, 1.08 mmol, 1.1 e.q) were added. The reaction mixture was reacted at 15°C for 0.5 hours. After TLC detection showed that the reaction was completed, the reaction liquid was extracted with DCM (30 mL × 2), and purified by column chromatography (eluent polarity : V_{Petroleum ether} : V_{Ethyl acetate} = 15 : 1 to 5 : 1) to obtain compound **16-3** as a reddish-brown oil (200 mg, yield: 99.0%).

Characterization data: ¹H NMR (400 MHz, CDCl₃) δ 8.29 (s, 1H), 7.19 (, *J* = 8.0 Hz, 1H), 6.96 (d, *J =* 8.0 Hz, 1H), 6.85 (t, *J* = 8.0 Hz, 1H), 4.14 (d, *J* = 8.0 Hz, 1H), 3.39 (m, 1H), 2.47 (s, 1H), 1.52 - 1.41 (m, 1H), 1.27 (d, *J* = 6.0 Hz, 6H), 0.74 - 0.66 (m, 2H), 0.48 - 0.41 (m, 2H).

### Step III: Synthesis of compound 16-4 (2-(cyclopropyl(methylthio)methyl)-6-isopropylphenol)

Compound **16-3** (200 mg, 0.97 mmol) was added to methanethiol (0.606 g, 10% propylene glycol solution, 1.26 mmol, 1.3 eq) under nitrogen protection, and concentrated hydrochloric acid (42.4 mg, 1.16 mmol, 1.2 eq) was added dropwise to the reaction liquid. The reaction system was stirred and reacted at 25°C for 4 hours. After TLC (V_{Hexane} : V_{EA} = 5 : 1) detection showed that a majority of raw materials were consumed, the reaction was stopped. The reaction liquid was diluted with H₂O and extracted with ethyl acetate (10 mL × 3). The organic phases were combined, washed with saturated saline and concentrated. The crude product was purified by column chromatography (eluent polarity : V_{n-Hexane} : V_{Ethyl acetate} = 50 : 1 to 20 : 1) to obtain compound **16-4** as a yellow oil (50 mg, yield: 21.8%).

Characterization data: ¹H NMR (400 MHz, CDCl₃) δ 7.56 (s, 1H), 7.20 - 7.19 (m, 1H), 6.98 - 6.95 (m, 1H), 6.89 - 6.85 (m, 1H), 3.42 - 3.38 (m, 1H), 3.22 - 3.19 (m, 1H), 1.94 (s, 3H), 1.48 - 1.41 (m, 1H), 1.28 - 1.25 (m, 6H), 0.79 - 0.73 (m, 1H), 0.65 - 0.60 (m, 1H), 0.46- 0.41 (m, 1H), 0.36 - 0.30 (m, 1H).

### Step IV: Synthesis of compound 16 (2-(cyclopropyl(methylsulfonyl)methyl)-6-isopropylphenol)

Under nitrogen protection, compound **16-4** (50 mg, 0.210 mmol) was dissolved in DCM (8 mL), and the system temperature was kept at -5°C-0°C. Meta-chloroperoxybenzoic acid (84.9 mg, 0.42 mmol, 2.0 eq) was slowly added to the reaction, and the reaction was stirred at 15°C for 0.5 hours. After TLC (V_{Hexane} : V_{EA} = 2 : 1) detection showed that the raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **16** as a yellow solid (20.1 mg, yield: 35.1%).

Characterization data: ¹H NMR (400 MHz, CDCl₃) δ 7.24 - 7.22 (m, 2H), 7.03 (t, *J* = 8.0 Hz, 1H), 6.85 - 6.82 (m, 1H), 3.72 (d, *J* = 8.0 Hz, 1H), 3.37 - 3.31 (m, 1H), 2.92 (s, 3H), 1.75 - 1.66 (m, 1H), 1.25 - 1.22 (m, 6H), 1.06 - 1.02 (m, 1H), 0.78 - 0.74 (m, 2H), 0.25 - 0.20 (m, 1H).

### Example 17

### Synthesis of compound 17 (2-(1-(methylsulfonyl)ethyl)-6-(pentan-3-yl)phenol)

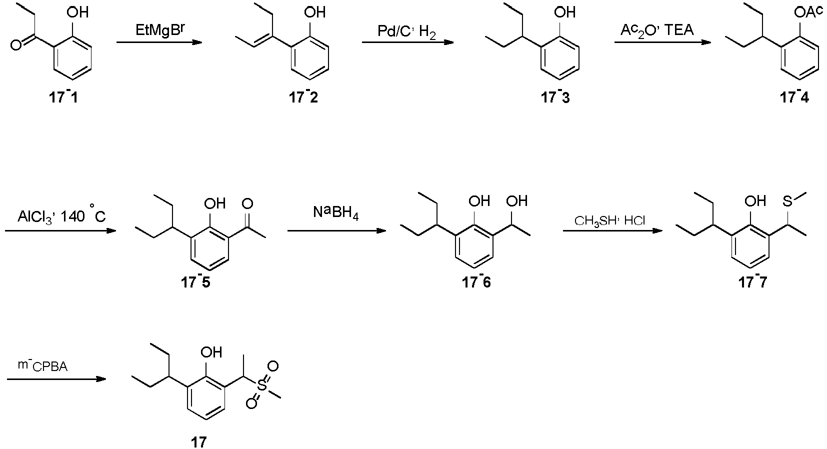

### Step I: Synthesis of compound 17-2 (2-(pentan-2-en-3-yl)phenol)

Compound **17-1** (5.0 g, 33.29 mmol, 1.0 eq) was added to a 200-mL three-necked flask under nitrogen protection, dry THF (60 mL) was added, and ethylmagnesium bromide (19.98 mL, 2.0 M in THF, 39.95 mmol, 1.2 e.q) was added slowly to the reaction at -15°C. The reaction mixture was reacted at -15°C for 2 hours. After TLC detection showed that the reaction was completed, the reaction liquid was diluted with ethyl acetate, and subjected to sample stirring and column separation (eluent : V_{Petroleum ether} : V_{Ethyl acetate} = 100 : 1 to 50 : 1) to obtain compound **17-2** as a yellow oil (4.0 g, yield: 74.6%).

Characterization data: ¹H NMR (400 MHz, CDCl₃) δ 7.63 (s, 1H), 7.24 - 7.16 (m, 2H), 6.97 - 6.94 (m, 1H), 6.76 - 6.73 (m, 1H), 6.06 - 6.00 (m, 1H), 2.06 - 2.00 (m, 5H), 1.06-1.02 (m, 3H).

### Step II: Synthesis of compound 17-3 (2-(pentan-3-yl)phenol)

Under hydrogen 103.42 kPa (15 Psi) protection, compound **17-2** (4.0 g, 24.66 mmol, 1.0 eq) was added to a 250-mL single-necked flask, dry methanol (60 mL) was added, and palladium on carbon (800 mg, Pd: 10%) was added to the reaction at 15°C. The reaction mixture was reacted in hydrogen 103.42 kPa (15 Psi) at 15°C for 12 hours. After TLC detection showed that the reaction was completed, the reaction liquid was filtered through celite, washed with methanol, dried and concentrated to obtain compound **17-3** as a grey oil (4.0 g, yield: 95.0%).

Characterization data: ¹H NMR (400 MHz, CDCl₃) δ 7.67 (s, 1H), 7.24 - 7.16 (m, 2H), 6.97 - 6.94 (m, 1H), 6.76 - 6.73 (m, 1H), 2.45 - 2.42 (m, 1H), 2.06 - 2.00 (m, 4H), 1.06 -1.02 (m, 6H).

### Step III: Synthesis of compound 17-4 (2-(pentan-3-yl)phenyl acetate)

Compound **17-3** (4.0 g, 24.35 mmol, 1.0 eq) was added to a 250-mL single-necked flask under nitrogen protection, dry DCM (60 mL) was added, and triethylamine (3.7 g, 36.53 mmol, 1.5 e.q.) and acetic anhydride (2.98 g, 29.22 mmol, 1.2 e.q.) were added to the reaction at 15°C. The reaction mixture was reacted at 15°C for 12 hours. After TLC detection showed that the reaction was completed, the reaction liquid was neutralized with dilute hydrochloric acid to pH = 6-7, washed with saturated saline, dried over anhydrous sodium sulphate, and concentrated to obtain compound **17-4** as a colourless oil (4.5 g, yield: 89.6%).

Characterization data: ¹H NMR (400 MHz, CDCl₃) δ 7.24 -7.16 (m, 2H), 6.97 - 6.94 (m, 1H), 6.76-6.73 (m, 1H), 2.45 - 2.42 (m, 1H), 2.36 (s, 3H), 2.06 - 2.00 (m, 4H), 1.06-1.02 (m, 6H).

### Step IV: Synthesis of compound 17-5 (1-(2-hydroxyl-3-(pentan-3-yl)phenyl)ethan-1-one)

Compound **17-4** (4.5 g, 21.81 mmol, 1.0 eq) was added to a 100-mL single-necked flask under nitrogen protection, dry DCM (60 mL) was added, and aluminium trichloride (3.78 g, 28.36 mmol, 1.3 e.q.) was added in portions to the reaction at 15°C. The reaction mixture was heated to 140°C and reacted for 4 hours. After TLC detection showed that the reaction was completed, the reaction liquid was extracted with DCM. The organic phase was washed with saturated saline, dried and concentrated. The crude product was purified by column chromatography to obtain compound **17-5** as a yellow oil (2.26 g, yield: 50.1%).

¹H NMR (400 MHz, CDCl₃) δ7.37 (s, 1H), 7.24 - 7.16 (m, 1H), 6.97 - 6.94 (m, 1H), 6.76 - 6.73 (m, 1H), 2.45 - 2.42 (m, 1H), 2.18 (s, 3H), 2.06 - 2.02 (m, 4H), 1.08 - 1.04 (m, 6H).

### Step V: Synthesis of compound 17-6 (2-(1-hydroxyethyl)-6-(pentan-3-yl)phenol)

Compound **17-5** (2.0 g, 9.70 mmol, 1.0 eq) was added to dry methanol (30 mL) under nitrogen protection, and sodium borohydride (0.440 g, 11.61 mmol, 1.2 e.q.) was added in portions to the reaction at 15°C. The reaction mixture was reacted at 20°C for 1 hour. After TLC detection showed that the reaction was completed, the reaction liquid was extracted with DCM. The organic phase was washed with saturated saline, dried and concentrated to obtain compound 17-6 as a yellow oil (2.01 g, yield: 99.0%).

Characterization data: ¹H NMR (400 MHz, CDCl₃) δ 7.37 (s, 1H), 7.24 - 7.17 (m, 1H), 6.97 - 6.94 (m, 1H), 6.76 - 6.73 (m, 1H), 2.54 (s, 1H), 2.45 - 2.42 (m, 1H), 2.06 - 1.98 (m, 4H), 1.91 (s, 3H), 1.24 - 1.17 (m, 6H).

### Step VI: Synthesis of compound 17-7 (2-(1-(methylthio)ethyl)-6-(pentan-3-yl)phenol)

Compound **17-6** (2.0 g, 9.60 mmol) was added to methanethiol (5.54 g, 10% propylene glycol solution, 11.52 mmol, 1.2 eq) under nitrogen protection, and hydrochloric acid (420 mg, 11.52 mmol, 1.2 eq) was added dropwise to the reaction liquid. The reaction system was stirred and reacted at 15°C for 12 hours. After TLC (V_{Hexane} : V_{EA} = 5 : 1) detection showed that a majority of raw materials were consumed, the reaction was stopped. The reaction liquid was diluted with H₂O and extracted with ethyl acetate (100 mL × 3). The organic phases were combined, washed with saturated saline and concentrated. The crude product was purified by column chromatography (eluent polarity : V_{n-Hexane} : V_{Ethyl acetate} = 80 : 1 to 25 : 1) to obtain compound **17-7** as a light yellow oil (1.1 g, yield: 48.1%).

Characterization data: ¹H NMR (400 MHz, CDCl₃) δ 7.63 (s, 1H), 7.26 - 7.21 (m, 1H), 7.12 - 7.06 (m, 1H), 6.96 - 6.86 (m, 1H), 4.05 - 4.00 (m, 1H), 2.45 - 2.42 (m, 1H), 2.06 - 1.98 (m, 4H), 1.94 (s, 3H), 1.63 (d, *J* = 8.0 Hz, 3H), 1.21 - 1.14 (m, 6H).

### Step VII: Synthesis of compound 17 (2-(1-(methylsulfonyl)ethyl)-6-(pentan-3-yl)phenol)

Under nitrogen protection, compound **17-7** (400 mg, 1.68 mmol) was dissolved in DCM (12 mL), and the system temperature was kept at -5°C-0°C. Meta-chloroperoxybenzoic acid (681.3 mg, 3.36 mmol, 2.0 eq) was slowly added to the reaction, and the reaction was conducted at 15°C for 0.5 hours. After TLC (V_{Hexane} : V_{EA} = 2 : 1) detection showed that the raw materials were consumed, the reaction was stopped. The reaction liquid was diluted with dichloromethane (30 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 1 : 1) to obtain compound **17** as a yellow oil (100 mg, yield: 22.0%).

Characterization data: ¹H NMR (400 MHz, CDCl₃) δ 7.65 (s, 1H), 7.26 -7.18 (m, 1H), 7.12 - 7.06 (m, 1H), 6.96 - 6.86 (m, 1H), 4.62 - 4.58 (m, 1H), 2.78 (s, 3H), 2.35 - 2.30 (m, 1H), 2.06 - 1.98 (m, 4H), 1.83 (d, *J* = 8.0 Hz, 3H), 1.21 - 1.14 (m, 6H).

### Example 18

### Synthesis of compound 18 (2-(1-cyclopropylethyl)-6-(1-(ethylsulfonyl)propyl)phenol)

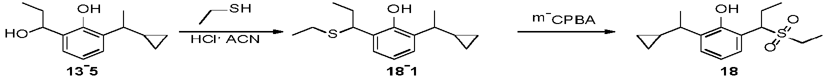

### Step I: Synthesis of compound 18-1 (2-(1-cyclopropylethyl)-6-(1-(ethylthio)propyl)phenol)

Compound **13-5** (200 mg, 0.91 mmol) was added to acetonitrile (5 mL), and ethanethiol (67.7 mg, 1.09 mmol, 1.2 eq) was added under nitrogen protection. Then hydrochloric acid (36.41 mg, 0.998 mmol, 1.1 eq) was added dropwise to the reaction liquid. The reaction system was stirred and reacted at 25°C for 6 hours. After TLC (V_{Hexane} : V_{EA} = 10 : 1) detection showed that a majority of raw materials were consumed, the reaction was stopped. The reaction liquid was diluted with H₂O and extracted with ethyl acetate (20 mL × 3). The organic phases were combined, washed with saturated saline and concentrated. The crude product was purified by column chromatography (eluent polarity : V_{n-Hexane} : V_{Ethyl acetate} = 1 : 0 to 100 : 1) to obtain compound **18-1** as a yellow oil (210 mg, yield: 87.5%).

Characterization data: ¹H NMR (400 MHz, CDCl₃) δ 7.58 (d, *J =* 4.0 Hz, 1H), 7.26 - 7.24 (m, 1H), 6.86 - 6.80 (m, 2H), 3.90 (d, *J* = 8.0 Hz, 1H), 2.58 - 2.53 (m, 1H), 2.34 - 2.28 (m, 2H), 1.99 - 1.88 (m, 2H), 1.30 (dd, *J* = 8.0 , 4.0 Hz, 3H), 1.16 - 1.12 (m, 3H), 1.04-0.98 (m, 1H), 0.96 (t, *J* = 8.0 Hz, 3H), 0.55 - 0.51 (m, 1H), 0.39 - 0.33 (m, 1H), 0.21-0.13 (m, 2H).

### Step II: Synthesis of compound 18 (2-(1-cyclopropylethyl)-6-(1-(ethylsulfonyl)propyl)phenol):

Under nitrogen protection, compound **18-1** (200 mg, 0.820 mmol) was dissolved in DCM (10 mL), and the system temperature was kept at -5°C-0°C. Meta-chloroperoxybenzoic acid (306 mg, 1.64 mmol, 2.0 eq) was slowly added to the reaction, and the reaction was conducted at 15°C for 0.5 hours. After TLC (V_{Hexane} : V_{EA} = 2 : 1) detection showed that the raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 15 : 1) to obtain compound **18** as a yellow oil (200 mg, yield: 89.3%).

Characterization data: ¹H NMR (400 MHz, CDCl₃) δ 7.34 - 7.31 (m, 1H), 7.03 - 7.00 (m, 1H), 6.97 - 6.93 (m, 1H), 4.47 - 4.05 (m, 1H), 2.90 - 2.83 (m, 2H), 2.64 - 2.53 (m, 1H), 2.46 - 2.28 (m, 2H), 1.31 - 1.27 (m, 6H), 1.05 - 0.98 (m, 1H), 0.92 (t, *J =* 8.0 Hz, 3H), 0.59 - 0.52 (m, 1H), 0.43 - 0.40 (m, 1H), 0.23 - 0.10 (m, 2H).

### Example 19

### Synthesis of compound 19 (2-(1-cyclopropylethyl)-6-(1-((2,2,2-trifluoroethyl)sulfonyl)ethyl)phenol)

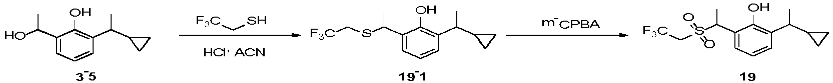

### Step I: Synthesis of compound 19-1 (2-(1-cyclopropylethyl)-6-(1-((2,2,2-trifluoroethyl)thio)ethyl)phenol)

Compound **3-5** (1.0 g, 4.85 mmol) was added to acetonitrile (10 mL), and trifluoroethylthiol (674 mg, 5.81 mmol, 1.2 eq) was added under nitrogen protection. Then hydrochloric acid (229 mg, 6.30 mmol, 1.3 eq) was added dropwise to the reaction liquid. The reaction system was stirred and reacted at 15°C for 10 hours. After TLC (V_{Hexane} : V_{EA} = 5 : 1) detection showed that a majority of raw materials were consumed, the reaction was stopped. The reaction liquid was diluted with H₂O and extracted with ethyl acetate (50 mL × 3). The organic phases were combined, washed with saturated saline and concentrated. The crude product was purified by column chromatography (eluent polarity : V_{n-Hexane} : V_{Ethyl acetate} = 50 : 1 to 20 : 1) to obtain compound **19-1** as a yellow oil (800 mg, yield: 65.7%).

Characterization data: ¹H NMR (400 MHz, CDCl₃) δ 7.50 (d, *J* = 8.0 Hz, 1H), 7.27 - 7.25 (m, 1H), 6.93 - 6.91 (m, 1H), 6.86 - 6.83 (m, 1H), 4.16 - 4.08 (m, 1H), 2.93 - 2.82 (m, 2H), 2.58 - 2.52 (m, 1H), 1.64 (d, *J* = 8.0 Hz, 3H), 1.31 - 1.28 (m, 3H), 1.05 - 0.97 (m, 1H), 0.59 - 0.54 (m, 1H), 0.38 - 0.33 (m, 1H), 0.20 - 0.16 (m, 2H).

### Step II: Synthesis of compound 19 (2-(1-cyclopropylethyl)-6-(1-((2,2,2-trifluoroethyl)sulfonyl)ethyl)phenol):

Under nitrogen protection, compound **19-1** (200 mg, 0.657 mmol) was dissolved in DCM (10 mL), and the system temperature was kept at -5°C - 0°C. Meta-chloroperoxybenzoic acid (265.9 mg, 1.314 mmol, 2.0 eq) was slowly added to the reaction, and the reaction was conducted at 15°C for 0.5 hours. After TLC (V_{Hexane} : V_{EA} = 1 : 1) detection showed that the raw materials were consumed, the reaction was stopped. The reaction liquid was diluted with dichloromethane (10 mL), and washed with a saturated sodium bicarbonate solution. The organic phases were combined, washed with saturated saline, dried over anhydrous sodium sulphate, filtered and concentrated. The crude product was purified by column chromatography (eluent polarity : V_{n-Hexane} : V_{Ethyl acetate} = 15 : 1) to obtain compound **19** as a colourless oil (185.0 mg, yield: 83.7%).

Characterization data: ¹H NMR (400 MHz, CDCl₃) δ 7.63 - 7.59 (m, 1H), 7.32 - 7.27 (m, 2H), 7.10 - 7.06 (m, 1H), 4.75 - 4.68 (m, 1H), 3.94 - 3.88 (m, 2H), 2.56 - 2.54 (m, 1H), 1.92 (d, *J* = 8.0 Hz, 3H), 1.35 - 1.24 (m, 3H), 1.05 - 1.00 (m, 1H), 0.59 - 0.54 (m, 1H), 0.42 - 0.40 (m, 1H), 0.28 - 0.16 (m, 2H).

### Example 20

### Synthesis of compound 20 (2-((R)-1-cyclopropylethyl)-6-((S)-1-(ethylsulfonyl)ethyl)phenol)

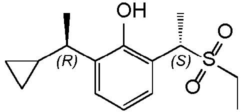

Characterization data: ¹H NMR (400 MHz, Chloroform-d) δ 7.36 (dd, J = 7.6, 1.7 Hz, 1H), 7.12 (dd, J = 7.7, 1.7 Hz, 1H), 7.10 (s, 1H), 7.00 (t, J = 7.7 Hz, 1H), 4.60 (q, J = 7.3 Hz, 1H), 2.93 (q, J = 7.5 Hz, 2H), 2.65 - 2.53 (m, 1H), 1.85 (d, J = 7.2 Hz, 3H), 1.38 - 1.28 (m, 6H), 1.08-0.99 (m, 1H), 0.62 - 0.56 (m, 1H), 0.49 - 0.38 (m, 1H), 0.28 - 0.22 (m, 1H), 0.20 - 0.14 (m, 1H).

### Example 21

### Synthesis of compound 21 (2-((S)-1-cyclopropylethyl)-6-((R)-1-(ethylsulfonyl)ethyl)phenol)

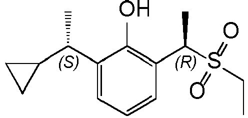

Characterization data: ¹H NMR (400 MHz, Chloroform-d) δ 7.36 (d, J = 7.6 Hz, 1H), 7.13 (d, J = 8.0 Hz, 1H),7.11 (s, 1H), 7.01 (t, J = 7.7 Hz, 1H), 4.60 (q, J = 7.3 Hz, 1H), 2.94 (q, J = 7.4 Hz, 2H), 2.69 - 2.52 (m, 1H), 1.85 (d, J = 7.4 Hz, 3H), 1.36 - 1.30 (m, 6H), 1.09-1.00 (m, 1H), 0.62 - 0.56 (m, 1H), 0.49 - 0.37 (m, 1H), 0.26 - 0.21 (m, 1H), 0.20 - 0.15 (m, 1H).

### Example 22

### Synthesis of compound 22 (2-((S)-1-cyclopropylethyl)-6-((S)-1-(ethylsulfonyl)ethyl)phenol)

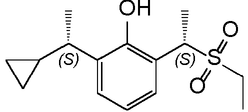

Characterization data: ¹H NMR (400 MHz, Chloroform-d) δ 7.37 (dd, J = 7.6, 1.7 Hz, 1H), 7.13 (dd, J = 7.7, 1.7 Hz, 1H),7.11 (s, 1H), 7.00 (t, J = 7.7 Hz, 1H), 4.60 (q, J = 7.3 Hz, 1H), 2.93 (q, J = 7.5 Hz, 2H), 2.74 - 2.46 (m, 1H), 1.85 (d, J = 7.3 Hz, 3H), 1.41 - 1.25 (m, 6H), 1.10 - 0.97 (m, 1H), 0.62 - 0.56 (m, 1H), 0.47 - 0.40 (m, 1H), 0.28 - 0.22 (m, 1H), 0.20 - 0.14 (m, 1H).

### Example 23

### Synthesis of compound 23 (2-((R)-1-cyclopropylethyl)-6-((R)-1-(ethylsulfonyl)ethyl)phenol)

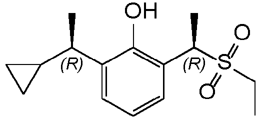

Characterization data: ¹H NMR (400 MHz, Chloroform-d) δ 7.36 (dd, J = 7.7, 1.7 Hz, 1H), 7.13 (dd, J = 7.8, 1.6 Hz, 1H), 7.11 (s, 1H), 7.00 (t, J = 7.7 Hz, 1H), 4.60 (q, J = 7.3 Hz, 1H), 2.93 (q, J = 7.5 Hz, 2H), 2.66 - 2.54 (m, 1H), 1.85 (d, J = 7.3 Hz, 3H), 1.39 - 1.23 (m, 6H), 1.08 - 1.00 (m, 1H), 0.62 - 0.56 (m, 1H), 0.47 - 0.41 (m, 1H), 0.27 - 0.21 (m, 1H), 0.20 - 0.14 (m, 1H).

**Preparation method of Examples 20-23:** Chiral isomer 2-(1-cyclopropylethyl)-6-(1-(ethylsulfonyl)ethyl)phenol (compound **11**) (1.5 g, 5.3 mmol) was subjected to chiral resolution by an HPLC method with preparation equipment and a chiral column.

Separation conditions: chiral column: CHIRALCEL OJ-H, mobile phase: V_{n-Hexane} : V_{Ethanol} = 90 : 10, flow rate: 25 mL/min, UV = 214 nm, and column temperature: 35°C.

Components with retention times of 6.908 min, 10.044 min and 11.829 min were collected respectively, and concentrated under reduced pressure,
wherein
(1) 660 mg of oil with retention time of 6.908 min was obtained as a pair of enantiomers; resolution was conducted again, and components with retention times of 8.831 min and 10.374 min were collected respectively, and concentrated under reduced pressure:
   ① compound **20** (350 mg, a white solid, HPLC purity: 99.05%, Chiral-HPLC purity: 100.00%, yield: 23.3%) with retention time of 8.831 min was obtained;
   ② compound **21** (290 mg, a white solid, HPLC purity: 99.47%, Chiral-HPLC purity: 99.94%, yield: 19.3%) with retention time of 10.374 min was obtained;
(2) compound **22** (354 mg, a white solid, HPLC purity: 97.10%, Chiral-HPLC purity: 99.88%, yield: 23.6%) with retention time of 10.044 min was obtained;
(3) compound **23** (322 mg, a white solid, HPLC purity: 98.43%, Chiral-HPLC purity: 99.53%, yield: 21.5%) with retention time of 11.829 min was obtained.

### Example 24 Biological test example

### Experiment I Righting reflex experiment on mice

SPF-grade ICR mice, each weighing 18-22 g, half male and half female, were used. A well-established mouse anesthesia model was used to study the general anesthesia effect of the compound of the present invention. The compounds were formulated with solvents of 10% DMSO, 15% solutol (HS15) and 75% saline to the desired concentration, for further use. After adapting to the experimental environment, the experimental animals were fasted with water available for 12 h, and were administered via intravenous injection at an administration volume of 10 mL/kg. Then the anesthesia induction time (the time period from drug administration to disappearance of the righting reflex) and the anesthesia maintenance time (the time period from disappearance of the righting reflex to recovery of the righting reflex) were recorded. Indicators including the median effective dose (ED₅₀), median lethal dose (LD₅₀), therapeutic index (TI, i.e. LD₅₀/ED₅₀), anesthesia induction time, anesthesia maintenance time and maximum tolerated dose were used to evaluate the effect and safety of anesthesia. Among them, control group 1 (propofol), control group 2 (ciprofol, a racemate), and control group 3 (ciprofol, R-configuration) respectively had the following structural formulas. The specific experimental results were shown in Table 1.

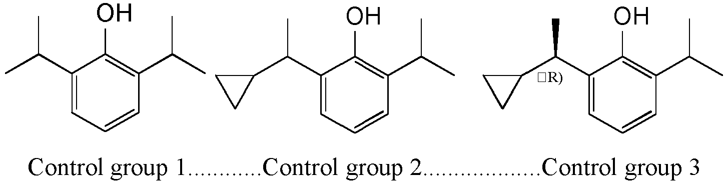

**Table 1 Data from righting reflex experiment on mice**

| Compound No. | ED₅₀ (mg/kg) | LD₅₀ (mg/kg) | TI | Anesthesia induction time (s) | Anesthesia maintenance time (s) | MTD (mg/kg) |
|---|---|---|---|---|---|---|
| Control group 1 | 12 | 31 | 2.6 | 19 | 586 | 20 |
| Control group 2 | 3.5 | 17 | 4.9 | 2 | 534 | 9 |
| Control group 3 | 1.2 | 6 | 5 | 2 | 521 | 3.1 |
| Compound 1 | 8 | 35 | 4.4 | 60 | 255 | 24 |
| Compound 2 | 6 | 17 | 3.8 | 2 | 690 | 10 |
| Compound 4 | 2 | 10 | 5.0 | 2 | 356 | 4 |
| Compound 5 | 6 | 10 | 1.7 | 2 | 1638 | 8 |
| Compound 6 | 11 | 34 | 3.1 | 5 | 579 | 22 |
| Compound 11 | 2 | 11 | 5.5 | 22 | 560 | 6 |
| Compound 14 | 4 | 12 | 3.0 | 2 | 499 | 9 |
| Compound 20 | 0.6 | 3.5 | 5.8 | 2 | 573 | 1.8 |
| Compound 21 | 2.5 | 6 | 2.4 | 2 | 354 | 3.5 |
| Compound 22 | 5 | 18 | 3.6 | 2 | 384 | 9 |
| Compound 23 | 7 | 14 | 2 | 2 | 294 | 10 |

Experimental conclusion: compared with control group 1, control group 2 and control group 3, the compound of the present invention showed higher therapeutic index and safety coefficient, and had a broader therapeutic window. The compound of the present invention had lower ED₅₀ value, indicating that the compound had a lower minimum effective dose and higher activity. The compound of the present invention showed a low free concentration in the aqueous phase of corresponding formulations, and was expected to have an effect of avoiding pain on injection.

### Experiment II Righting reflex experiment on rats

SD rats, half male and half female, were fasted with water available for 12 h, and were administered via intravenous injection at an administration volume of 10 mL/kg. Then the anesthesia induction time (the time period from drug administration to disappearance of the righting reflex), the anesthesia maintenance time (the time period from disappearance of the righting reflex to recovery of the righting reflex and revival) and the anesthesia recovery time (the time period from revival to complete recovery) were recorded. Indicators including the median effective dose (ED₅₀), median lethal dose (LD₅₀), therapeutic index (TI, i.e. LD₅₀/ED₅₀), anesthesia induction time, anesthesia maintenance time and maximum tolerated dose were used to evaluate the effect and safety of anesthesia.

**Table 2 Data from righting reflex experiment on rats**

| Compound No. | ED₅₀ (mg/kg) | LD₅₀ (mg/kg) | TI | Anesthesia induction time (s) | Anesthesia maintenance time (s) | MTD (mg/kg) |
|---|---|---|---|---|---|---|
| Control group 3 | 1.1 | 5.6 | 5.1 | 34 | 409 | 4 |
| Compound 20 | 0.5 | 3.2 | 6.4 | 10 | 624 | 2.2 |

Experimental conclusion: compared with control group 3, compound 20 of the present invention showed higher therapeutic index and safety coefficient, and had a broader therapeutic window. Compound 20 of the present invention had lower ED₅₀ value, indicating that the compound had a lower minimum effective dose than that of control group 3 and higher activity.

### Experiment III Detection of the effect of the compound of the present invention on stably overexpressed GABA_{A} (α1β2γ2) receptor current using patch-clamp technique

Gamma-aminobutyric acid (GABA) was an important inhibitory amino acid neurotransmitter in the central nervous system, and functioned by binding to GABA receptors. The GABA receptors were divided into three subtypes: GABA_{A}, GABA_{B} and GABA_{C}, among which the GABA_{A} receptor was the most important. The GABA_{A} receptor was an anion-selective ion channel that could enhance chloride ion permeability and thus reduce neuronal excitability. The GABA_{A} receptor was involved in regulating general anesthesia and was also closely related to diseases such as neurological and psychiatric disorders, e.g., depression, insomnia, anxiety and epilepsy. A whole-cell patch-clamp technique was used to study the allosteric regulation effect of the compound of the present invention on the GABA_{A} (α1β2γ2) receptor.

HEK293 cell lines stably expressing the GABA_{A} (α1β2γ2) receptor were used. The GABA_{A} (α1β2γ2) receptor gene information was as follows: GABA-α1: NM_000806; GABA-β2: NM_021911; GABA-γ2: NM_198904. The voltage stimulus of GABA receptor current recorded by a whole-cell patch-clamp technique was as follows: when whole-cell sealing was formed, the cell membrane voltage was clamped at -70 mV. The peak value of current was recorded after sequentially spraying test compounds from low concentration to high concentration and 100 µM GABA onto the cell surface in a Gap-free mode. The mode of administration of test compounds was as follows: for each concentration, the test compound was administered 1-2 times; the cells were washed with extracellular fluid for 1 min before detection was performed on the test compound at another concentration; and finally, 100 µM GABA was given as the control. The experimental data was collected by an EPC-10 amplifier (HEKA) and stored in PatchMaster (HEKA) software. A microelectrode puller was used to pull capillary glass tubes into a recording electrode. A microelectrode manipulator was manipulated under an inverted microscope to contact the recording electrode with cells, and negative pressure suction was applied to form a GΩ seal. After the GΩ seal was formed, rapid capacitance compensation (pF) was conducted, and then negative pressure was continued to break cell membranes, forming a whole-cell recording mode. Then slow capacitance compensation was conducted, and the membrane capacitance (pF) and series resistance were recorded. No electric leakage compensation was provided. The cover glass spread with cells was placed in a recording bath of the inverted microscope, and a working solution of the test compound and the extracellular fluid without the compound sequentially flowed through the recording bath from low concentration to high concentration by gravity perfusion to act on the cells. During the recording, a vacuum pump was used for liquid exchange. Multiple cells were independently and repeatedly detected. All electrophysiological experiments were conducted at room temperature.

The allosteric regulation effect of the compound of the present invention on the GABA_{A} (α1β2γ2) receptor was detected in three independent repeated experiments, and the semi-activated concentration (EC₅₀) of the sample on the GABA_{A} (α1β2γ2) receptor was calculated by means of fitting. The experimental results were as follows:

**Table 3 Allosteric regulation effect of test compounds on GABA_{A} (α1β2γ2) receptor**

| Compound No. | n | EC₅₀ |
|---|---|---|
| Control group 3 | 3 | 10.40 µM |
| Compound 20 | 3 | 1.556 µM |

Experimental conclusion: the semi-activated concentration (EC50) of compound 20 of the present invention having allosteric regulation on GABA_{A} (α1β2γ2) was 1.556 µM, and the EC₅₀ of control group 3 was 10.40 µM. The results indicated that compared with control group 3, compound 20 of the present invention had a stronger allosteric regulation effect on GABA_{A} (α1β2γ2), and could exert anesthesia effects by agonizing GABA_{A} (α1β2γ2).

### Experiment IV Pharmacokinetic study of the compound of the present invention in rats

Twelve male SD rats (200-300 g) were randomized into 2 groups (n = 6): control group 3 and compound 20. The rats were administered via intravenous injection at 1 mg/kg (control group 3) and 1 mg/kg (compound 20), respectively, with an administration volume of 5 mL/kg and a vehicle of 5% DMSO+10% solutol (HS15)+85% Saline. Blood samples were taken before administration and at 2 min, 4 min, 8 min, 12 min, 15 min, 30 min, 1 h, 1.5 h and 2 h after administration. Plasma samples were taken by centrifugation, and stored in a -80°C refrigerator until LC-MS analysis to be tested. After sample processing, quantitative analysis of substances in plasma was conducted using LCMS/MS to detect the concentration of prototype compounds in the plasma. The plasma concentration/time curve obtained in this way was used to calculate pharmacokinetic parameters by a validated pharmacokinetic computer program. The experimental results were shown in Table 4.

**Table 4 Pharmacokinetic parameters of rats after intravenous administration**

| Compound No. | Mode of administration | T_{1/2} (h) | AUClast (h*ng/mL) |
|---|---|---|---|
| Control group 3 | iv | 0.843 | 142 |
| Compound 20 | iv | 0.33 | 195.5 |

Experimental conclusion: the pharmacokinetic properties of compound 20 of the present invention were similar to those of control group 3. Metabolism clearance in rats was faster, which was consistent with the anesthesia effect. After anesthesia, the rats could wake up quickly, while avoiding toxicity accumulation in the bodies, suggesting that the compound had good pharmacokinetic properties.

### Experiment V Pharmacokinetic study of the compound of the present invention in Beagle dogs

Six male Beagle dogs (6-10 kg) were randomized into 2 groups (n = 3): control group 3 and compound 20. The rats were administered via intravenous injection at 0.5 mg/kg (control group 3) and 0.5 mg/kg (compound 20), respectively, with an administration volume of 1 mL/kg and a vehicle of 5% DMSO+10% solutol (HS15)+85% Saline. Blood samples were taken before administration and at 2 min, 5 min, 10 min, 20 min, 30 min, 1 h, 1.5 h, 2 h and 3 h after administration. Plasma samples were taken by centrifugation, and stored in a - 80°C refrigerator until LC-MS analysis to be tested. After sample processing, quantitative analysis of substances in plasma was conducted using LCMS/MS to detect the concentration of prototype compounds in the plasma. The plasma concentration/time curve obtained in this way was used to calculate pharmacokinetic parameters by a validated pharmacokinetic computer program. The experimental results were shown in Table 5.

**Table 5 Pharmacokinetic parameters of Beagle dogs after intravenous administration**

| Compound No. | Mode of administration | T_{1/2} (h) | AUClast (h*ng/mL) |
|---|---|---|---|
| Control group 3 | iv | 0.63 | 144.8 |
| Compound 20 | iv | 0.79 | 127.8 |

Experimental conclusion: the pharmacokinetic properties of compound 20 of the present invention were similar to those of control group 3. Metabolism clearance in Beagle dogs was faster, and toxicity accumulation in the bodies was avoided, suggesting that the compound had good pharmacokinetic properties.

### Experiment VI Hemodynamic effect study of the compound of the present invention in Beagle dogs

The experiment used an emka PACK 4G telemetry system to detect the effects of intravenous injection of control group 3 and compound 20 on electrocardiogram, blood pressure and body temperature in conscious Beagle dogs, providing reference information for evaluating the safety of clinical use.

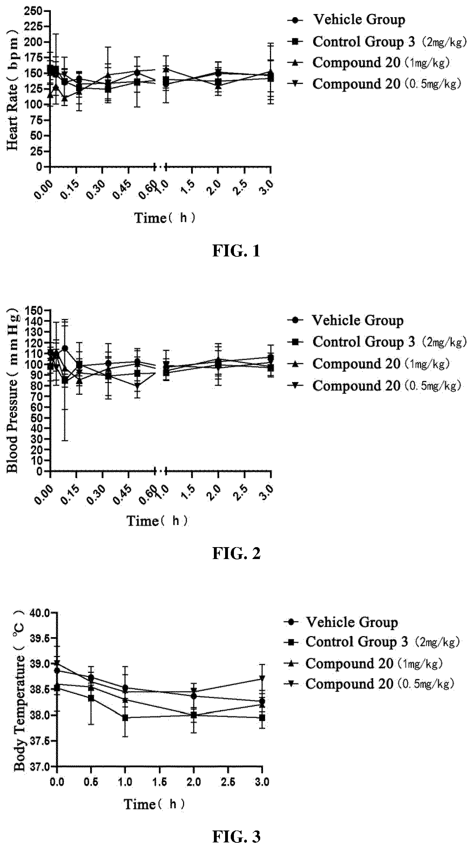

Four Beagle dogs were subjected to crossover administration, with a vehicle control (5% DMSO+10% solutol+85% Saline), control group 3 (2 mg/kg), compound 20 (0.5 mg/kg) and compound 20 (1 mg/kg) for each intravenous injection, with an administration volume of 2 mL/kg. Observations were respectively conducted before administration and within 1 h after administration on the day of administration. Anal temperature was measured before administration and at 30 min, 1 h, 2 h and 3 h after administration during each administration period; and electrocardiogram parameters were continuously collected from 0.5 h before administration to 3 h after administration during each administration period. Blood pressure was collected at 15 min after collection was started, and was collected at 2 min, 5 min, 10 min, 20 min, 30 min, 1 h, 2 h and 3 h after administration. The electrocardiogram, blood pressure data, body temperature, and clinical observation results on the day of administration at various time points including before administration (at 30 min after collection was started) and at 2 min, 5 min, 10 min, 20 min, 30 min, 1 h, 2 h and 3 h after administration were analysed and evaluated. The experimental results were shown in FIG. 1, FIG. 2 and FIG. 3.

Experimental conclusion: the experimental results showed a slow down in heart rate of animals within 1 h after administration of control group 3 and compound 20, and a recovery in heart rate after 1 h. After administration, although all indicators of blood pressure of animals in each group decreased, the amplitude was not significant, among which the systolic pressure decreased unapparently, and was still within the normal range. After administration, there were no abnormalities in the body temperature indicator of animals in each group. The results indicated that compound 20 of the present invention had little effect on the heart rate, blood pressure and body temperature of Beagle dogs, and therefore, it was expected that compound 20 of the present invention had good safety.

## Claims

1. A compound as represented by general formula (I), or a stereoisomer or pharmaceutically acceptable salt thereof: 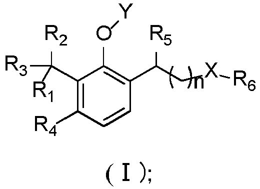 wherein,
X is selected from the group consisting of 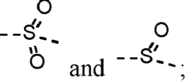
R₁ is selected from the group consisting of C₁₋₆ alkyl, C₁₋₆ alkene, C₁₋₆ alkyne, 3- to 6-membered heterocycloalkyl and 3- to 6-membered cycloalkyl, wherein the alkyl, alkene, alkyne, heterocycloalkyl and cycloalkyl may be optionally further substituted with one or more R;
R₂ and R₃ are independently selected from the group consisting of H, hydroxyl, F, C₁₋₆ alkyl, C₁₋₆ alkene, C₁₋₆ alkyne, 3- to 6-membered heterocycloalkyl, C₁₋₆ alkoxy, CN, NH₂ and 3- to 6-membered cycloalkyl, wherein the alkyl, alkene, alkyne, heterocycloalkyl, alkoxy and cycloalkyl may be optionally further substituted with one or more R;
or alternatively, R₂ and R₃ may form (=O);
R₄ is selected from the group consisting of H, F, Cl, Br, I, hydroxyl, C₁₋₆ alkyl, C₁₋₆ alkoxy, 3- to 5-membered cycloalkyl and 3- to 5-membered heterocyclyl;
or alternatively, R₁ and R₄ together with the atoms to which they are attached form 4- to 6-membered cycloalkyl or heterocyclyl fused to a benzene ring, wherein the cycloalkyl and heterocyclyl may be optionally further substituted with one or more R;
R₅ is selected from the group consisting of C₂-C₅ alkenyl, C₂-C₅ alkynyl, C₁₋₆ alkyl and 3- to 6-membered cycloalkyl, wherein the alkyl and cycloalkyl may be optionally further substituted with one or more R;
R₆ is selected from the group consisting of C₁₋₆ alkyl, 3- to 6-membered cycloalkyl and NHR₇, wherein the alkyl and cycloalkyl may be optionally further substituted with one or more R;
R₇ is C₁₋₆ alkyl or cycloalkyl;
Y is selected from the group consisting of H, Na, K, 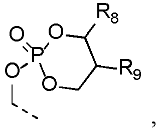 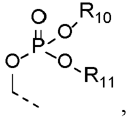 -(CH₂) ₘCOOR₁₂, 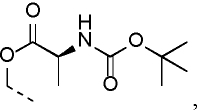 **and** C₁₋₁₀ alkyl, wherein the alkyl is optionally further substituted with one or more R;
R₈ and R₉ are each independently selected from the group consisting of H, C₁₋₆ alkyl, C₁₋₆ alkoxy, F, Cl, Br, I, hydroxyl, amino, cyano and carboxyl;
or alternatively, R₈ and R₉ together with the atoms to which they are attached form a 5- to 8-membered ring, wherein the 5- to 8-membered ring may contain 0 to 4 heteroatoms selected from the group consisting of N, O and S;
R₁₀ and R₁₁ are each independently selected from the group consisting of H, C₁₋₆ alkyl, an alkaline metal ion, an alkaline earth metal ion, a protonated amine and a protonated amino acid, wherein the alkaline metal ion is selected from the group consisting of Na⁺, K⁺ and Li⁺, the alkaline earth metal ion is selected from the group consisting of Be²⁺, Mg²⁺ and Ca²⁺, the amine is selected from the group consisting of trometamol, triethanolamine, ethanolamine, triethylamine and N-methylglucosamine, and the amino acid is arginine or lysine;
R₁₂ is independently selected from the group consisting of H, C₁₋₆ alkyl, 3- to 8-membered cycloalkyl and 4- to 8-membered heterocyclyl, wherein the alkyl, cycloalkyl and heterocyclyl may be optionally further substituted with one or more R;
R is selected from the group consisting of F, Cl, Br, I, deuterium, hydroxyl, carboxyl, CN, NH₂, C₁₋₆ alkyl, C₁₋₆ alkoxy, 3- to 5-membered cycloalkyl and 3- to 5-membered heterocyclyl;
n is 0, 1, 2 or 3; and
m is 0, 1, 2, 3 or 4.

2. The compound or the stereoisomer or pharmaceutically acceptable salt thereof according to claim 1, **characterized in that** the compound is a compound as represented by general formula (II): 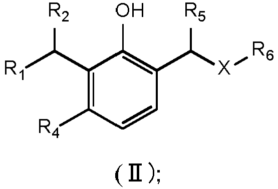 wherein X is selected from the group consisting of

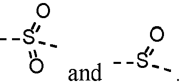

3. The compound or the stereoisomer or pharmaceutically acceptable salt thereof according to claim 1, **characterized in that** the compound is a compound as represented by general formula (III): 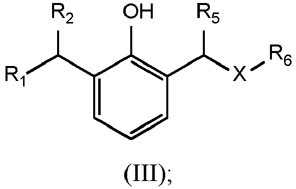 wherein
X is selected from the group consisting of 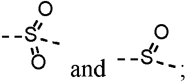 and
R₁, R₂, R₅ and R₆ are each independently C₁₋₆ alkyl or 3- to 6-membered cycloalkyl.

4. The compound or the stereoisomer or pharmaceutically acceptable salt thereof according to claim 2, **characterized in that**
R₁ is C₁₋₆ alkyl or 3- to 6-membered cycloalkyl;
R₂ is selected from the group consisting of H, hydroxyl, C₁₋₆ alkyl, C₁₋₆ alkoxy and 3- to 6-membered cycloalkyl;
R₅ is C₁₋₆ alkyl or 3- to 6-membered cycloalkyl; and
R₆ is C₁₋₆ alkyl or 3- to 6-membered cycloalkyl, wherein the alkyl and cycloalkyl may be optionally further substituted with one or more R.

5. The compound or the stereoisomer or pharmaceutically acceptable salt thereof according to claim 2, wherein
R₂ is selected from the group consisting of H, hydroxyl, C₁₋₆ alkyl, C₁₋₆ alkoxy and 3- to 6-membered cycloalkyl;
R₁ and R₄ together with the atoms to which they are attached form 4- to 6-membered cycloalkyl or heterocyclyl fused to a benzene ring, wherein the cycloalkyl and heterocyclyl may be optionally further substituted with one or more R;
R₅ is C₁₋₆ alkyl or 3- to 6-membered cycloalkyl; and
R₆ is C₁₋₆ alkyl or 3- to 6-membered cycloalkyl.

6. The compound or the stereoisomer or pharmaceutically acceptable salt thereof according to claim 4, wherein
R₂ is independently C₁₋₆ alkyl or 3- to 6-membered cycloalkyl;
R₄ is H;
R₆ is C₁₋₆ alkyl or 3- to 6-membered cycloalkyl, wherein the alkyl and cycloalkyl may be optionally further substituted with one or more R; and
R is selected from the group consisting of F, Cl, Br and I.

7. The compound or the stereoisomer or pharmaceutically acceptable salt thereof according to claim 5, wherein
R₁ and R₄ together with the atoms to which they are attached form 4- to 6-membered cycloalkyl fused to a benzene ring, wherein the cycloalkyl may be optionally further substituted with one or more R; and
R is C₁₋₆ alkyl or C₁₋₆ alkoxy.

8. The compound or the stereoisomer or pharmaceutically acceptable salt thereof according to claim 1, wherein the compound is selected from the group consisting of:

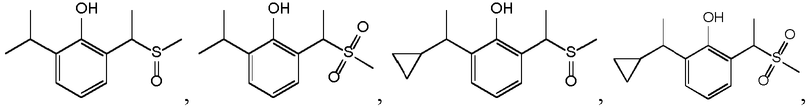

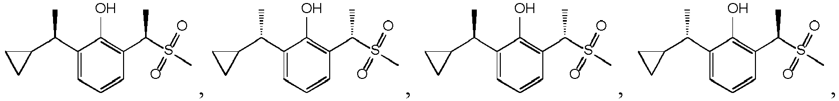

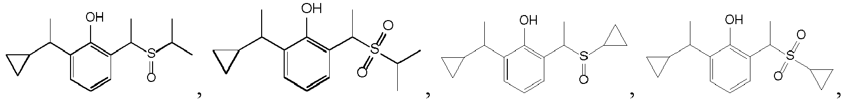

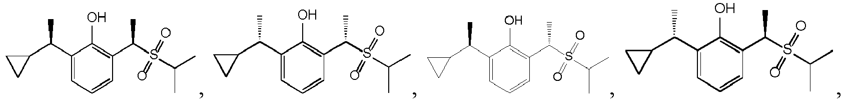

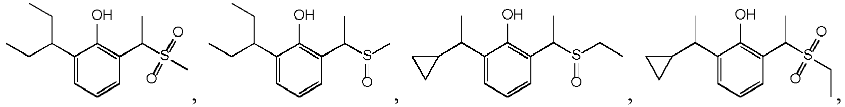

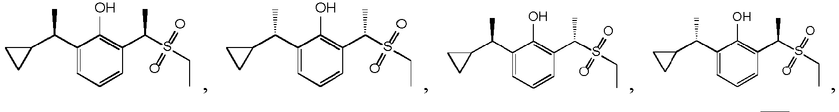

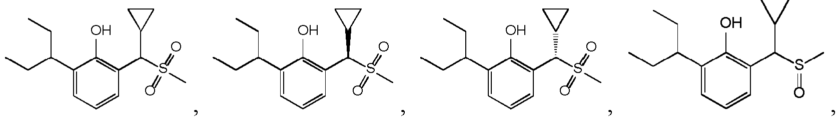

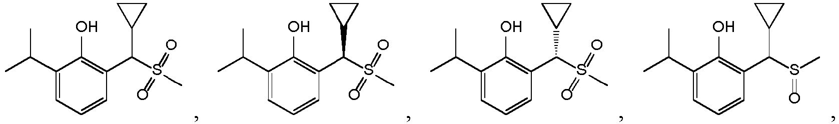

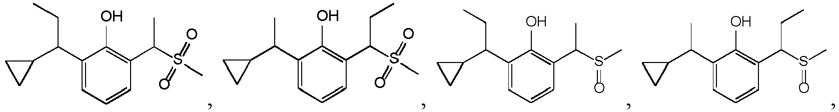

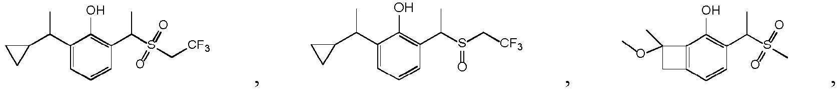

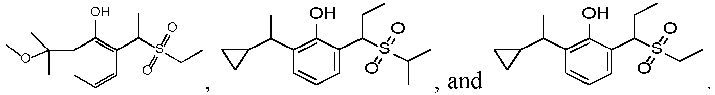

9. A pharmaceutical composition, comprising the compound or the stereoisomer or pharmaceutically acceptable salt thereof according to any one of claims 1 to 8, and one or more pharmaceutically acceptable carriers.

10. Use of the compound or the stereoisomer or pharmaceutically acceptable salt thereof according to any one of claims 1 to 8 or the pharmaceutical composition according to claim 9 in the preparation of a medicament for inducing and/or maintaining anesthesia in an animal or human body, facilitating sedation and hypnosis in an animal or human body, and treating and/or preventing anxiety, depression, insomnia, nausea, vomiting, migraine, schizophrenia, convulsion and epilepsy.

## Patentansprüche

1. Verbindung der allgemeinen Formel (I) oder Stereoisomer bzw. pharmazeutisch akzeptables Salz davon, 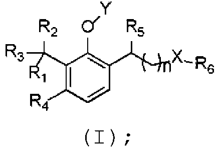 worin
X aus der Gruppe bestehend aus 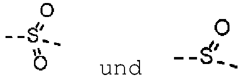 ausgewählt ist;
R₁ aus der Gruppe bestehend aus C₁₋₆-Alkyl-, C₁₋₆-Alkenyl-, C₁₋₆-Alkinyl-, 3- bis 6-gliedriger Heterocycloalkyl- und 3- bis 6-gliedriger Cycloalkylgruppe ausgewählt ist, wobei die Alkyl-, Alkenyl-, Alkinyl-, Heterocycloalkyl- und Cycloalkylgruppe optional weiter mit einem oder mehreren R substituiert sein können;
R₂ und R₃ voneinander unabhängig aus der Gruppe bestehend aus H, Hydroxyl-, F, C₁₋₆-Alkyl-, C₁₋₆-Alkenyl-, C₁₋₆-Alkinyl-, 3- bis 6-gliedriger Heterocycloalkyl-, C₁₋₆-Alkoxy-, CN, NH₂ und 3- bis 6-gliedriger Cycloalkylgruppe ausgewählt ist, wobei die Alkyl-, Alkenyl-, Alkinyl-, Heterocycloalkyl-, Alkoxy- und Cycloalkylgruppe optional weiter mit einem oder mehreren R substituiert sein können;
oder alternativ R₂ und R₃ zusammen (=O) bilden können;
R₄ aus der Gruppe bestehend aus H, F, Cl, Br, I, Hydroxyl-, C₁₋₆-Alkyl-, C₁₋₆-Alkoxyl-, 3- bis 5-gliedriger Cycloalkyl- und 3- bis 5-gliedriger Heterocyclylgruppe ausgewählt ist;
oder alternativ R₁ und R₄ zusammen mit den Atomen, an die sie gebunden sind, eine 4- bis 6-gliedrige Cycloalkylgruppe oder Heterocyclylgruppe, die mit einem Benzolring fusioniert ist, bilden, wobei die Cycloalkylgruppe und Heterocyclylgruppe optional weiter mit einem oder mehreren R substituiert sein können;
R₅ aus der Gruppe bestehend aus C₂₋₅-Alkenyl-, C₂₋₅-Alkinyl-, C₁₋₆-Alkyl- und 3- bis 6-gliedriger Cycloalkylgruppe ausgewählt ist, wobei die Alkylgruppe und Cycloalkylgruppe optional weiter mit einem oder mehreren R substituiert sein können;
R₆ aus der Gruppe bestehend aus C₁₋₆-Alkylgruppe, 3- bis 6-gliedriger Cycloalkylgruppe und NHR₇ ausgewählt ist, wobei die Alkylgruppe und Cycloalkylgruppe optional weiter mit einem oder mehreren R substituiert sein können;
R₇ C₁₋₆-Alkylgruppe oder Cycloalkylgruppe ist;
Y aus der Gruppe bestehend aus H, Na, K, 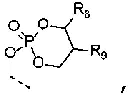 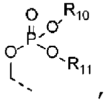 -(CH₂)ₘCOOR₁₂, 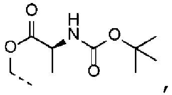 und C₁₋₁₀-Alkylgruppe ausgewählt ist, wobei die Alkylgruppe optional weiter mit einem oder mehreren R substituiert ist;
R₈ und R₉ voneinander unabhängig aus der Gruppe bestehend aus H, C₁₋₆-Alkyl-, C₁₋₆-Alkoxy-, F, Cl, Br, I, Hydroxyl-, Amino-, Cyano- und Carboxylgruppe ausgewählt sind;
oder alternativ R₈ und R₉ zusammen mit den Atomen, an die sie gebunden sind, einen 5- bis 8-gliedrigen Ring bilden, wobei der 5- bis 8-gliedrige Ring 0 bis 4 Heteroatome ausgewählt aus der Gruppe bestehend aus N, O und S enthalten kann;
R₁₀ und R₁₁ voneinander unabhängig aus der Gruppe bestehend aus H, C₁₋₆-Alkylgruppe, Alkalimetallion, Erdalkalimetallion, protoniertem Amin und protonierter Aminosäure ausgewählt sind, wobei die Alkalimetallion aus der Gruppe bestehend aus Na⁺, K⁺ und Li⁺ ausgewählt ist, die Erdalkalimetallion aus der Gruppe bestehend aus Be²⁺, Mg²⁺ und Ca²⁺ ausgewählt ist, das Amin aus der Gruppe bestehend aus Trometamol, Triethanolamin, Ethanolamin, Triethylamin und N-Methylglukosamin ausgewählt ist, und die Aminosäure aus der Gruppe bestehend aus Arginin und Lysin ausgewählt ist;
R₁₂ voneinander unabhängig aus der Gruppe bestehend aus H, C₁₋₆-Alkyl-, 3- bis 8-gliedriger Cycloalkyl- und 4-bis 8-gliedriger Heterocyclylgruppe ausgewählt ist, wobei die Alkylgruppe, Cycloalkylgruppe und Heterocyclylgruppe optional weiter mit einem oder mehreren R substituiert sein können;
R aus der Gruppe bestehend aus F, Cl, Br, I, Deuterium, Hydroxyl-, Carboxyl-, CN, NH₂, C₁₋₆-Alkyl-, C₁₋₆-Alkoxyl-, 3- bis 5-gliedriger Cycloalkyl- und 3- bis 5-gliedriger Heterocyclylgruppe ausgewählt ist,
n 0, 1, 2 oder 3 ist; und
m 0, 1, 2, 3 oder 4 ist.

2. Verbindung der allgemeinen Formel (I) oder Stereoisomer bzw. pharmazeutisch akzeptables Salz davon nach Anspruch 1, **dadurch gekennzeichnet, dass** die Verbindung eine Verbindung der allgemeinen Formel (II) ist: 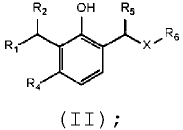 worin X aus der Gruppe bestehend aus 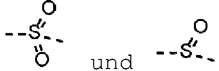 ausgewählt ist.

3. Verbindung der allgemeinen Formel (I) oder Stereoisomer bzw. pharmazeutisch akzeptables Salz davon nach Anspruch 1, **dadurch gekennzeichnet, dass** die Verbindung eine Verbindung der allgemeinen Formel (III) ist: 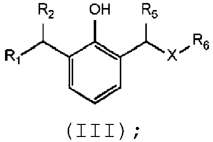 worin
X aus der Gruppe bestehend aus 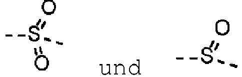 ausgewählt ist; und
R₁, R₂, R₅ und R₆ jeweils voneinander unabhängig C₁₋₆-Alkylgruppe oder 3- bis 6-gliedriger Cycloalkylgruppe sind.

4. Verbindung der allgemeinen Formel (I) oder Stereoisomer bzw. pharmazeutisch akzeptables Salz davon nach Anspruch 2, **dadurch gekennzeichnet, dass**
R₁ C₁₋₆-Alkylgruppe oder 3- bis 6-gliedriger Cycloalkylgruppe ist;
R₂ aus der Gruppe bestehend aus H, Hydroxyl-, C₁₋₆-Alkyl-, C₁₋₆-Alkoxy-, und 3- bis 6-gliedriger Cycloalkylgruppe ausgewählt ist;
R₅ C₁₋₆-Alkylgruppe oder 3- bis 6-gliedriger Cycloalkylgruppe ist; und
R₆ C₁₋₆-Alkylgruppe oder 3- bis 6-gliedriger Cycloalkylgruppe ist, wobei die Alkylgruppe und Cycloalkylgruppe optional weiter mit einem oder mehreren R substituiert sein können.

5. Verbindung der allgemeinen Formel (I) oder Stereoisomer bzw. pharmazeutisch akzeptables Salz davon nach Anspruch 2, worin
R₂ aus der Gruppe bestehend aus H, Hydroxyl-, C₁₋₆-Alkyl-, C₁₋₆-Alkoxy-, und 3- bis 6-gliedriger Cycloalkylgruppe ausgewählt ist;
R₁ und R₄ zusammen mit den Atomen, an die sie gebunden sind, eine 4- bis 6-gliedrige Cycloalkylgruppe oder Heterocyclylgruppe, die mit einem Benzolring fusioniert ist, bilden, wobei die Cycloalkylgruppe und Heterocyclylgruppe optional weiter mit einem oder mehreren R substituiert sein können;
R₅ C₁₋₆-Alkylgruppe oder 3- bis 6-gliedriger Cycloalkylgruppe ist; und
R₆ C₁₋₆-Alkylgruppe oder 3- bis 6-gliedriger Cycloalkylgruppe ist.

6. Verbindung der allgemeinen Formel (I) oder Stereoisomer bzw. pharmazeutisch akzeptables Salz davon nach Anspruch 4, worin
R₂ voneinander unabhängig C₁₋₆-Alkylgruppe oder 3- bis 6-gliedriger Cycloalkylgruppe ist;
R₄ H ist;
R₆ C₁₋₆-Alkylgruppe oder 3- bis 6-gliedriger Cycloalkylgruppe ist, wobei die Alkylgruppe und Cycloalkylgruppe optional weiter mit einem oder mehreren R substituiert sein können; und
R aus der Gruppe bestehend aus F, Cl, Br und I ausgewählt ist.

7. Verbindung der allgemeinen Formel (I) oder Stereoisomer bzw. pharmazeutisch akzeptables Salz davon nach Anspruch 5, worin
R₁ und R₄ zusammen mit den Atomen, an die sie gebunden sind, eine 4- bis 6-gliedrige Cycloalkylgruppe, die mit einem Benzolring fusioniert ist, bilden, wobei die Cycloalkylgruppe optional weiter mit einem oder mehreren R substituiert sein kann; und
R C₁₋₆-Alkylgruppe oder C₁₋₆-Alkoxygruppe ist.

8. Verbindung der allgemeinen Formel (I) oder Stereoisomer bzw. pharmazeutisch akzeptables Salz davon nach Anspruch 1, wobei die Verbindung ausgewählt ist aus der Gruppe bestehend aus:

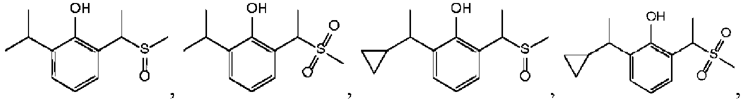

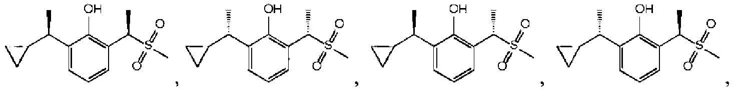

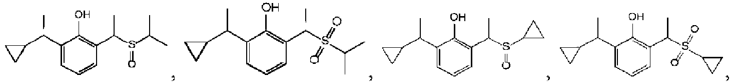

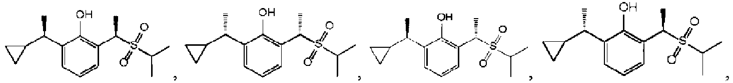

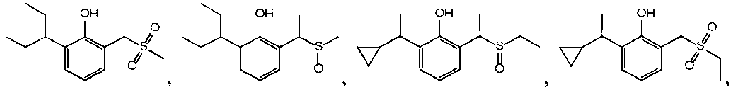

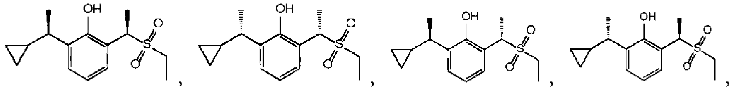

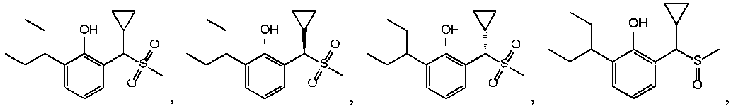

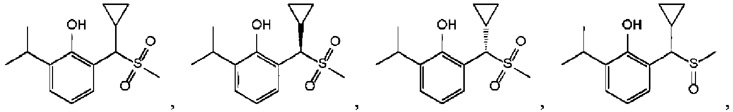

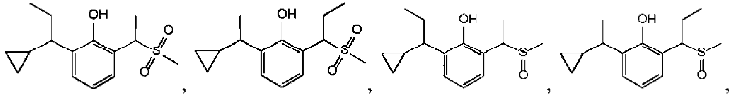

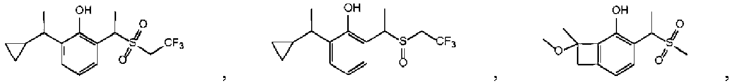

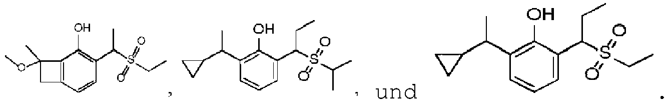

9. Pharmazeutische Zusammensetzung, umfassend die Verbindung oder das Stereoisomer bzw. pharmazeutisch akzeptable Salz davon gemäß einem der Ansprüche 1 bis 8 und einen oder mehrere pharmazeutisch akzeptable Träger.

10. Verwendung der Verbindung oder des Stereoisomers bzw. pharmazeutisch akzeptablen Salzes davon gemäß einem der Ansprüche 1 bis 8 oder der pharmazeutischen Zusammenfassung gemäß dem Anspruch 9 in Herstellung von Arzneimitteln für Einführung und/oder Aufrechterhaltung von Anästhesie in einem tierischen oder menschlichen Körper, für Unterstützung von Sedierung und Hypnose in einem tierischen oder menschlichen Körper, und für Behandlung und/oder Prävention von Angst, Depression, Schlaflosigkeit, Brechreiz, Emesis, Migräne, Schizophrenie, Krampf und Epilepsie.

## Revendications

1. Composé représenté par la formule générale (I), ou un stéréoisomère ou un sel pharmaceutiquement acceptable de celui-ci: 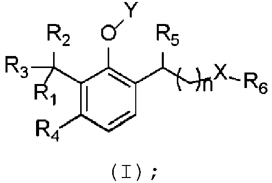 dans lequel,
X est choisi dans le groupe constitué de 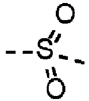 et 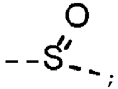
R₁ est choisi dans le groupe constitué d'un alkyle en C₁₋₆, d'un alcényle en C₁₋₆, d'un alcynyle en C₁₋₆, d'un hétérocycloalkyle à 3 à 6 chaînons et d'un cycloalkyle à 3 à 6 chaînons, dans lequel l'alkyle, l'alcényle, l'alcynyle, l'hétérocycloalkyle et le cycloalkyle peuvent être éventuellement en outre substitués par un ou plusieurs R;
R₂ et R₃ sont indépendamment choisis dans le groupe constitué de H, d'un hydroxyle, de F, d'un alkyle en C₁₋₆, d'un alcényle en C₁₋₆, d'un alcynyle en C₁₋₆, d'un hétérocycloalkyle à 3 à 6 chaînons, d'un alkoxy en C₁₋₆, de CN, de NH₂ et d'un cycloalkyle à 3 à 6 chaînons, dans lequel l'alkyle, l'alcényle, l'alcynyle, l'hétérocycloalkyle, l'alkoxy et le cycloalkyle peuvent être éventuellement en outre substitués par un ou plusieurs R;
ou alternativement, R₂ et R₃ peuvent former (=O);
R₄ est choisi dans le groupe constitué de H, de F, de Cl, de Br, de I, d'un hydroxyle, d'un alkyle en C₁₋₆, d'un alkoxy en C₁₋₆, d'un cycloalkyle à 3 à 5 chaînons et d'un hétérocycle à 3 à 5 chaînons;
ou alternativement, R₁ et R₄ forment ensemble avec les atomes auxquels ils sont attachés un cycloalkyle ou hétérocycle à 4 à 6 chaînons fusionné à un noyau benzénique, dans lequel le cycloalkyle et l'hétérocycle peuvent être éventuellement en outre substitués par un ou plusieurs R;
R₅ est choisi dans le groupe constitué d'un alcényle en C₂-C₅, d'un alcynyle en C₂-C₅, d'un alkyle en C₁₋₆ et d'un cycloalkyle à 3 à 6 chaînons, dans lequel l'alkyle et le cycloalkyle peuvent être éventuellement en outre substitués par un ou plusieurs R;
R₆ est choisi dans le groupe constitué d'un alkyle en C₁₋₆, d'un cycloalkyle à 3 à 6 chaînons et NHR₇, dans lequel l'alkyle et le cycloalkyle peuvent être éventuellement en outre substitués par un ou plusieurs R;
R₇ est un alkyle en C₁₋₆ ou un cycloalkyle;
Y est choisi dans le groupe constitué de H, Na, K, 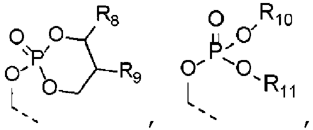 -(CH₂)ₘCOOR₁₂, 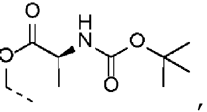 et d'un alkyle en C₁₋₁₀, dans lequel l'alkyle est éventuellement en outre substitué par un ou plusieurs R;
R₈ et R₉ sont chacun indépendamment choisis dans le groupe constitué de H, d'un alkyle en C₁₋₆, d'un alkoxy en C₁₋₆, de F, de Cl, de Br, de I, d'un hydroxyle, d'un amino, d'un cyano et d'un carboxyle;
ou alternativement, R₈ et R₉ forment ensemble avec les atomes auxquels ils sont attachés un cycle à 5 à 8 chaînons, dans lequel le cycle à 5 à 8 chaînons peut contenir 0 à 4 hétéroatomes choisis dans le groupe constitué de N, O et S;
R₁₀ et R₁₁ sont chacun indépendamment choisis dans le groupe constitué de H, d'un alkyle en C₁₋₆, d'un ion métal alcalin, d'un ion métal alcalino-terreux, d'une amine protonée et d'un acide aminé protoné, dans lequel l'ion métal alcalin est choisi dans le groupe constitué de Na⁺, K⁺ et Li⁺, l'ion métal alcalino-terreux est choisi dans le groupe constitué de Be²⁺, Mg²⁺ et Ca²⁺, l'amine est choisie dans le groupe constitué du trométamol, de la triéthanolamine, de l'éthanolamine, de la triéthylamine et de la N-méthylglucosamine, et l'acide aminé est l'arginine ou la lysine;
R₁₂ est indépendamment choisi dans le groupe constitué de H, d'un alkyle en C₁₋₆, d'un cycloalkyle à 3 à 8 chaînons et d'un hétérocycle à 4 à 8 chaînons, dans lequel l'alkyle, le cycloalkyle et l'hétérocycle peuvent être éventuellement en outre substitués par un ou plusieurs R;
R est choisi dans le groupe constitué de F, Cl, Br, I, du deutérium, d'un hydroxyle, d'un carboxyle, de CN, de NH₂, d'un alkyle en C₁₋₆, d'un alkoxy en C₁₋₆, d'un cycloalkyle à 3 à 5 chaînons et d'un hétérocycle à 3 à 5 chaînons;
n est 0, 1, 2 ou 3; et
m est 0, 1, 2, 3 ou 4.

2. Composé ou stéréoisomère ou sel pharmaceutiquement acceptable de celui-ci selon la revendication 1, **caractérisé en ce que** le composé est un composé représenté par la formule générale (II): 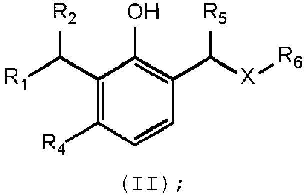 dans lequel X est choisi dans le groupe constitué de 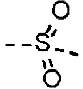 et

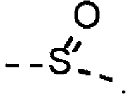

3. Composé ou stéréoisomère ou sel pharmaceutiquement acceptable de celui-ci selon la revendication 1, **caractérisé en ce que** le composé est un composé représenté par la formule générale (III): 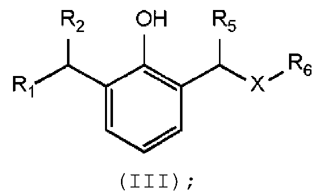 dans lequel
X est choisi dans le groupe constitué de 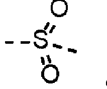 et 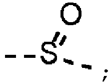 et
R₁, R₂, R₅ et R₆ sont chacun indépendamment un alkyle en C₁₋₆ ou un cycloalkyle à 3 à 6 chaînons.

4. Composé ou stéréoisomère ou sel pharmaceutiquement acceptable de celui-ci selon la revendication 2, **caractérisé en ce que**
R₁ est un alkyle en C₁₋₆ ou un cycloalkyle à 3 à 6 chaînons;
R₂ est choisi dans le groupe constitué de H, d'un hydroxyle, d'un alkyle en C₁₋₆, d'un alkoxy en C₁₋₆ et d'un cycloalkyle à 3 à 6 chaînons;
R₅ est un alkyle en C₁₋₆ ou un cycloalkyle à 3 à 6 chaînons; et
R₆ est un alkyle en C₁₋₆ ou un cycloalkyle à 3 à 6 chaînons, dans lequel l'alkyle et le cycloalkyle peuvent être éventuellement en outre substitués par un ou plusieurs R.

5. Composé ou stéréoisomère ou sel pharmaceutiquement acceptable de celui-ci selon la revendication 2, dans lequel
R₂ est choisi dans le groupe constitué de H, d'un hydroxyle, d'un alkyle en C₁₋₆, d'un alkoxy en C₁₋₆ et d'un cycloalkyle à 3 à 6 chaînons;
R₁ et R₄ forment ensemble avec les atomes auxquels ils sont attachés un cycloalkyle ou hétérocycle à 4 à 6 chaînons fusionné à un noyau benzénique, dans lequel le cycloalkyle et l'hétérocycle peuvent être éventuellement en outre substitués par un ou plusieurs R;
R₅ est un alkyle en C₁₋₆ ou un cycloalkyle à 3 à 6 chaînons; et
R₆ est un alkyle en C₁₋₆ ou un cycloalkyle à 3 à 6 chaînons.

6. Composé ou stéréoisomère ou sel pharmaceutiquement acceptable de celui-ci selon la revendication 4, dans lequel
R₂ est indépendamment un alkyle en C₁₋₆ ou un cycloalkyle à 3 à 6 chaînons;
R4 est H;
R₆ est un alkyle en C₁₋₆ ou un cycloalkyle à 3 à 6 chaînons, dans lequel l'alkyle et le cycloalkyle peuvent être éventuellement en outre substitués par un ou plusieurs R; et
R est choisi dans le groupe constitué de F, Cl, Br et I.

7. Composé ou stéréoisomère ou sel pharmaceutiquement acceptable de celui-ci selon la revendication 5, dans lequel
R₁ et R₄ forment ensemble avec les atomes auxquels ils sont attachés un cycloalkyle à 4 à 6 chaînons fusionné à un noyau benzénique, dans lequel le cycloalkyle peut être éventuellement en outre substitué par un ou plusieurs R; et
R est un alkyle en C₁₋₆ ou un alkoxy en C₁₋₆.

8. Composé ou stéréoisomère ou sel pharmaceutiquement acceptable de celui-ci selon la revendication 1, dans lequel le composé est choisi dans le groupe constitué de:

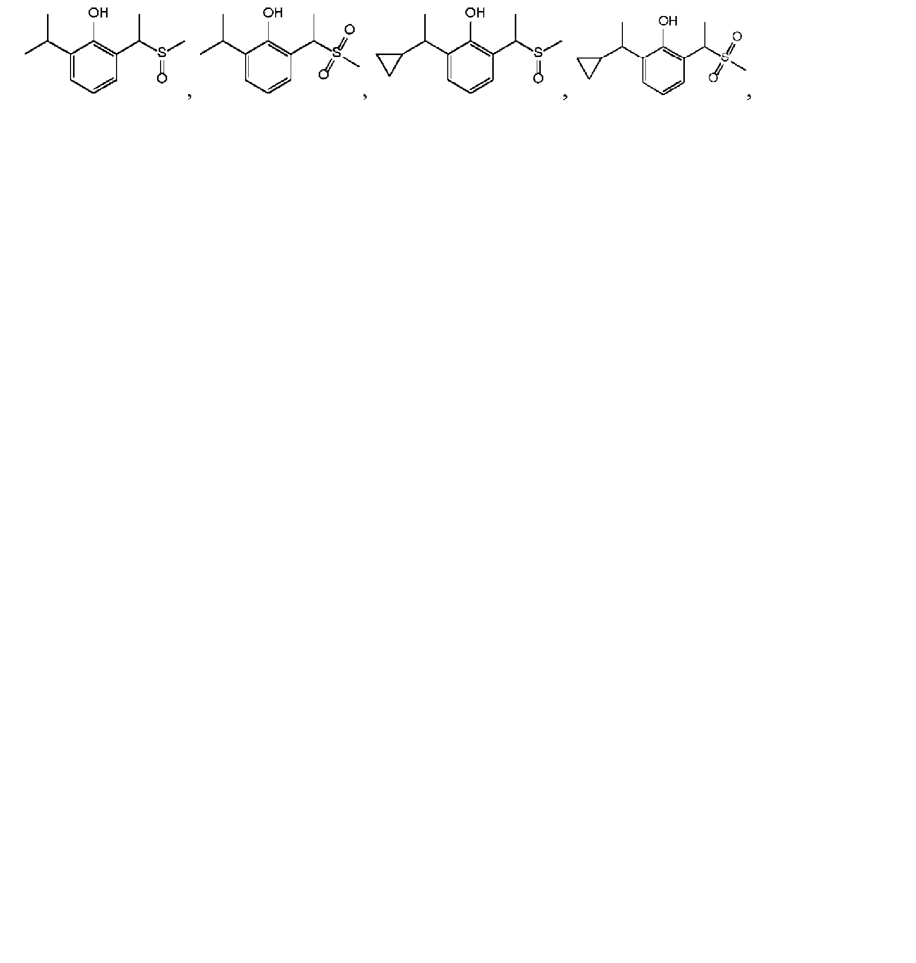

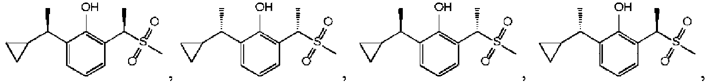

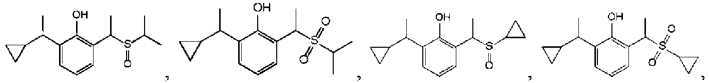

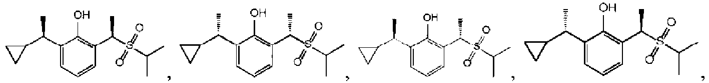

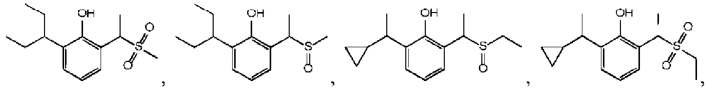

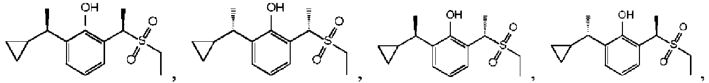

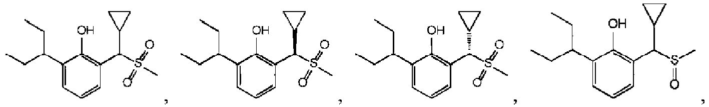

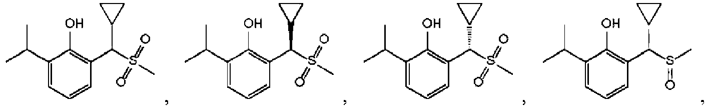

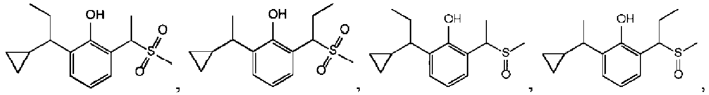

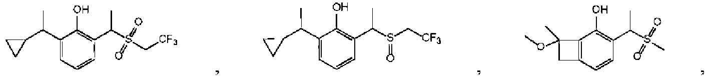

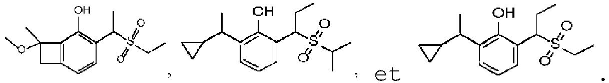

9. Composition pharmaceutique, comprenant le composé ou le stéréoisomère ou le sel pharmaceutiquement acceptable de celui-ci selon l'une quelconque des revendications 1 à 8, et un ou plusieurs excipients pharmaceutiquement acceptables.

10. Utilisation du composé ou du stéréoisomère ou du sel pharmaceutiquement acceptable de celui-ci selon l'une quelconque des revendications 1 à 8 ou de la composition pharmaceutique selon la revendication 9 dans la préparation d'un médicament pour induire et/ou maintenir une anesthésie chez un animal ou un corps humain, facilitant la sédation et l'hypnose chez un animal ou un corps humain, et traitant et/ou prévenant l'anxiété, la dépression, l'insomnie, la nausée, les vomissements, la migraine, la schizophrénie, la convulsion et l'épilepsie.
